# EUROPEAN PATENT APPLICATION

(11) **EP 2 090 311 A1**
(43) Date of publication of application: **19.08.2009**
(21) Application number: 08384001.7
(22) Date of filing: 18.02.2008
(51) Int. Cl.: A61K 31/495, A61P 25/02, A61P 29/02

(54) **Use of compounds binding to the sigma receptor ligands for the treatment of neuropathic pain developing as a consequence of chemotherapy**

(71) Applicant: Laboratorios Del. Dr. Esteve, S.A., 08041 Barcelona (ES)
(72) Inventor: Baeyens-Cabrera, José Manuel Dpt. Farmacia, F. Medicina, 18012 Granada (ES); Buschmann, Helmut H., 08960 Sant Just Desvern (ES); Vela Hernàndez, José Miguel, 08028 Barcelona (ES); Zamanillo-Castanedo, Daniel, 08041 Barcelona (ES); Nieto-López, Francisco Rafael Dpt. Farmacia, Facultad de Medicina, 18012 Granada (ES)
(74) Representative: Peters, Hajo

(57) **Abstract**

The present invention refers to the use of compounds binding to the sigma receptor for the treatment or prevention of neuropathic pain resulting from chemotherapy.

## Description

### Field of the invention

The present invention refers to the use of compounds binding to the sigma receptor for the treatment or prevention of neuropathic pain resulting from chemotherapy.

### Background of the invention

The treatment of pain conditions is of great importance in medicine. There is currently a world-wide need for additional pain therapy. The pressing requirement for a specific treatment of pain conditions or as well a treatment of specific pain conditions which is right for the patient, which is to be understood as the successful and satisfactory treatment of pain for the patients, is documented in the large number of scientific works which have recently and over the years appeared in the field of applied analgesics or on basic research on nociception.

PAIN is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210). Even though pain is always subjective, its causes or syndromes can be classified. The most relevant subtypes touched by this application are neuropathic pain, allodynia or hyperalgesia, especially also peripheral neuropathy.

On the other hand cancer and the therapy thereof are one of the biggest health concerns in the world. Besides surgery and close to always supplementing it chemotherapy is the method of choice for controlling or helping patients struck by carcinomas.

Chemotherapy is the use of chemical substances to treat disease and in the sense of this invention refers primarily to the use of cytotoxic drugs (called chemotherapeutic drugs) to treat cancer. Chemotherapy in cancer consists of a personalized combination of potent chemotherapy drugs, designed to slow rapid cancer tumor growth, shrink tumors, kill cancer cells, and prevent the spread of cancer. The chemotherapeutic drugs prevent cells from replicating in the typical, out-of-control manner in which cancer cells divide.

Peripheral neurotoxicity is a clinically significant complication of cancer chemotherapy. For several of the most effective drugs (e.g. taxanes, vincristine, cisplatin), neurotoxicity is dose-limiting and sometimes forces the termination of otherwise successful therapy (Polomano and Bennett, 2001). Since these drugs are the treatment of choice for a multitude of lymphoid and solid tumours, hundred of thousands of patients each year are affected. Sensory abnormalities from antineoplastic-evoked neurotoxicity range from mild paresthesiae or dysesthesiae in many patients to a chronic painful peripheral neuropathy in a subset (Quasthoff and Hartung, 2002). The occurrence and severity of the neuropathy is dependent on single dose intensity, duration of treatment, cumulative dose, prior or concurrent treatment with other neuropathic drugs and co-existing conditions such as diabetes and alcohol abuse (Alberts et al., 1995; Postma et al., 1995; Forsyth et al., 1997; Quasthoff and Hartung, 2002). Thus it is known that as a result of Chemotherapy in a considerable number of cases neuropathic pain/allodynia/hyperalgesia develops, especially as peripheral neuropathy. This is a very specific development of symptoms coming from neurotoxicity of the chemotherapeutic drug and treatment of that is crucial for assuring quality of live for patients suffering these consequences of chemotherapy.

Therefore, the objective of the present invention was to provide a new form of treatment for neuropathic pain/allodynia/hyperalgesia including peripheral neuropathy developing as a consequence of Chemotherapy.

It is surprising that administration of a compound binding to the sigma receptor, especially of a sigma receptor antagonist in form of a (neutral) antagonist, of an inverse agonist or a partial antagonist, is highly effective for the treatment for neuropathic pain/allodynia/hyperalgesia developing after Chemotherapy. Additionally it turned out to be even more surprising that co-treament of a compound binding to the sigma receptor together with chemotherapeutic drug was blocking even the development of pain often encountered as a consequence of Chemotherapy (as described above).

Thus, the present invention relates to the use of a compound binding to the sigma receptor for the production of a medicament for the treatment of pain developing as a consequence of Chemotherapy. Preferably thus the pain to be treated is either neuropathic pain, allodynia or hyperalgesia. If the pain is neuropathic pain it is selected from peripheral neuropathic pain, allodynia, causalgia, hyperalgesia, hyperesthesia, hyperpathia, neuralgia, neuritis or neuropathy.

In one preferred embodiment of the use according to the invention, the compound binding to the sigma receptor is used for the prevention of the development of pain developing as a consequence of chemotherapy.

In another preferred embodiment of the use according to the invention, the compound binding to the sigma receptor is used for the treatment of pain developing as a consequence of chemotherapy.

"Chemotherapy" in the sense of this invention is defined as the use of a chemotherapeutic drug for the treatment of cancer or tumors or malign neoplasia respectively.

"Developing as a consequence of Chemotherapy" according to this invention is defined as a) developing after or with the initiating of Chemotherapy and b) thus coinciding with or following the use of a chemotherapeutic drug. Therefore, the symptom to be treated - in all scientific likelihood - is being caused by or is due to the toxicity/citotoxicity, especially the (peripheral) neurotoxicity, of the chemotherapeutic drug.

"Chemotherapeutic drugs" in the sense of this invention are compounds used in chemotherapy, especially those working by impairing mitosis (cell division), effectively targeting fast-dividing cells. As these drugs cause damage to cells they are termed cytotoxic. Some drugs cause cells to undergo apoptosis (so-called "cell suicide"). Especially preferred chemotherapeutic drugs in the sense of this invention are drugs derived from platin, especially the platin-derivatives cisplatin, carboplatin and oxaliplatin; plant alkaloids and terpenes (terpenoids).

"Plant alkaloids" (and terpenoids) are alkoloids derived from plants and block cell division by preventing microtubule function. Microtubules are vital for cell division and without them it can not occur. The main examples are vinca alkaloids and taxanes.

"Vinca alkaloids" bind to specific sites on tubulin, inhibiting the assembly of tubulin into microtubules (M phase of the cell cycle). They are derived from the Madagascar periwinkle, *Catharanthus roseus* (formerly known as *Vinca rosea*). The vinca alkaloids include: Vincristine, Vinblastine, Vinorelbine, Vindesine.

"Taxanes" are derived from the Pacific yew tree, *Taxus brevifolia.* Taxanes enhance stability of microtubules, preventing the separation of chromosomes during anaphase. Taxanes include: Paclitaxel and Docetaxel.

Below is a list of chemotherapeutic drugs (by their trademarks), including paclitaxel (Taxol®), Iressa, Gefintinib and Xyotax:
13-cis-Retinoic Acid, 2-CdA, 2-Chlorodeoxyadenosine, 5-fluorouracil 5-FU, 6-Mercaptopurine, 6-MP, 6-TG 6-Thioguanine, Abraxane, Accutane ®, Actinomycin-D, Adriamycin ®, Adrucil ®, Agrylin ®, Ala-Cort ®, Aldesleukin, Alemtuzumab, ALIMTA, Alitretinoin, Alkaban-AQ ®, Alkeran ®, All-transretinoic acid, Alpha interferon, Altretamine, Amethopterin, Amifostine, Aminoglutethimide, Anagrelide, Anandron ®, Anastrozole, Arabinosylcytosine, Ara-C, Aranesp ®, Aredia ®, Arimidex ®, Aromasin ®, Arranon ®, Arsenic trioxide, Asparaginase, ATRA, Avastin ®, Azacitidine"BCG, BCNU, Bevacizumab, Bexarotene, BEXXAR ®, Bicalutamide, BiCNU, Blenoxane ®, Bleomycin, Bortezomib, Busulfan, Busulfex ®"C225, Calcium Leucovorin, Campath ®, Camptosar ®, Camptothecin-11, Capecitabine, Carac TM, Carboplatin, Carmustine, Carmustine wafer, Casodex ®, CC-5013, CCNU (o), CDDP (t), CeeNU (t), Cerubidine (t), cetuximab, Chlorambucil, Cisplatin, Citrovorum Factor, Cladribine, Cortisone, Cosmegen (t), CPT-11 (o), Cyclophosphamide, Cytadren (t), Cytarabine, Cytarabine liposomal, Cytosar-U (t), Cytoxan ®, Dacarbazine, Dactinomycin, Darbepoetin alfa, Daunomycin, Daunorubicin, Daunorubicin hydrochloride (t), Daunorubicin liposomal, DaunoXome (t), Decadron, Delta-Cortef (t), Deltasone (t), Denileukin, diftitox, DepoCyt (t), Dexamethasone, Dexamethasone acetate, dexamethasone sodium phosphate, Dexasone (t), Dexrazoxane, DHAD (o), DIC (t), Diodex (t), Docetaxel, Doxil (t), Doxorubicin, Doxorubicin liposomal, Droxia (t), DTIC, DTIC-Dome (t), Duralone (t), Efudex (t), Eligard (t), Ellence (t), Eloxatin (t), Elspar (t), Emcyt (t), Epirubicin, Epoetin alfa, Erbitux, Erlotinib, Erwinia L-asparaginase (t), Estramustine, Ethyol, Etopophos (t), Etoposide, Etoposide phosphate (t), Eulexin (t), Evista (t), Exemestane, Fareston (t), Faslodex (t), Femara ®, Filgrastim, Floxuridine, Fludara (t), Fludarabine, Fluoroplex (t), Fluorouracil, Fluorouracil (cream), Fluoxymesterone, Flutamide, Folinic Acid (o), FUDR (t), Fulvestrant, G-CSF (t), Gefitinib, Gemcitabine, Gemtuzumab ozogamicin, Gemzar (t), GleevecTM, Gliadel wafer (t), GM-CSF (o), Goserelin, granulocyte - colony stimulating factor (t), Granulocyte macrophage colony stimulating factor (o), Halotestin (t), Herceptin (t), Hexadrol (t), Hexalen (t), Hexamethylmelamine (t), HMM (t), Hycamtin (t), Hydrea (t), Hydrocort Acetate (t), Hydrocortisone, Hydrocortisone sodium phosphate, Hydrocortisone sodium succinate, Hydrocortone phosphate (t), Hydroxyurea, Ibritumomab, Ibritumomab Tiuxetan, Idamycin ®, Idarubicin Ifex ®, IFN-alpha, Ifosfamide, IL-11, IL-2, Imatinib mesylate, Imidazole Carboxamide, Interferon alfa, Interferon Alfa-2b (PEG conjugate) (o), Interleukin - 2 (t), Interleukin-11 (o), Intron A® (interferon alfa-2b), Iressa ®, Irinotecan, Isotretinoin, Kidrolase (t), Lanacort (t), L-asparaginase (t), LCR (o), Lenalidomide, Letrozole, Leucovorin, Leukeran (t), Leukine (t), Leuprolide, Leurocristine (o), Leustatin (t), Liposomal Ara-C (t), Liquid Pred (t), Lomustine, L-PAM (o), L-Sarcolysin (o), Lupron (t), Lupron Depot (t), Matulane (t), Maxidex (t), Mechlorethamine, Mechlorethamine Hydrochloride, Medralone (t), Medrol ®, Megace (t), Megestrol, Megestrol Acetate (o), Melphalan, Mercaptopurine, Mesna, Mesnex (t), Methotrexate, Methotrexate Sodium (o), Methylprednisolone, Meticorten (t), Mitomycin, Mitomycin-C (o), Mitoxantrone, M-Prednisol (t), MTC (o), MTX (o), Mustargen (t), Mustine, Mutamycin (t), Myleran (t), Mylocel (t), Mylotarg (t), Navelbine (t), Nelarabine, Neosar (t), Neulasta (t), Neumega (t), Neupogen (t), Nexavar ®, Nilandron (t), Nilutamide, Nipent ®, Nitrogen Mustard (o), Novaldex (t), Novantrone (t), Octreotide, Octreotide acetate (o), Oncospar (t), Oncovin (t), Ontak (t), Onxal (t), Oprevelkin, Orapred (t), Orasone (t), Oxaliplatin, Paclitaxel, Paclitaxel Protein-bound, Pamidronate, Panretin (t), Paraplatin (t), Pediapred (t), PEG Interferon, Pegaspargase, Pegfilgrastim, PEG-INTRON (t), PEG-L-asparaginase, PEMETREXED, Pentostatin, Phenylalanine Mustard (o), Platinol (t), Platinol-AQ (t), Prednisolone, Prednisone, Prelone (t), Procarbazine, PROCRIT ®, Proleukin (t), Prolifeprospan 20 with Carmustine implant (t), Purinethol (t), Raloxifene, Revlimid ®, Rheumatrex (t), Rituxan (t), Rituximab, Roferon-A®, (interferon alfa-2a) Rubex (t), Rubidomycin hydrochloride (t), Sandostatin ®, Sandostatin LAR (t), Sargramostim, Solu-Cortef (t), Solu-Medrol (t), Sorafenib, STI-571, Streptozocin, SU11248, Sunitinib, Sutent ®, Tamoxifen, Tarceva ®, Targretin (t), Taxol ®, Taxotere (t), Temodar ®, Temozolomide, Teniposide, TESPA (o), Thalidomide, Thalomid ®, TheraCys (t), Thioguanine, Thioguanine Tabloid (t), Thiophosphoamide (o), Thioplex (t), Thiotepa, TICE ®, Toposar (t), Topotecan, Toremifene, Tositumomab, Trastuzumab, Tretinoin, Trexall (t), Trisenox (t), TSPA (o), VCR (o), Velban (t), Velcade ®, VePesid (t), Vesanoid (t), Viadur (t), Vidaza (t), Vinblastine, Vinblastine Sulfate (o), Vincasar Pfs (t), Vincristine, Vinorelbine, Vinorelbine tartrate (o), VLB (o), VM-26 (o), VP-16 (t), Vumon (t), Xeloda ®, Xyotax, Zanosar (t), Zevalin TM, Zinecard (t), Zoladex ®, Zoledronic acid, Zometa ®.

In addition there is another list of drugs used in cancer-therapy (mostly as chemotherapeutics):
(as trademarks): Aldara, Alimta, Androcur, Arimidex, Borea, Caelyx, Campto, Casodex, Decapeptyl, Eloxatin, Eutirox, Faslodex, Femara, Gemzar, Gonapeptyl, Grisetin, Herceptin, Isovorin, Lysodren, Megefren, Metvix, Navelbine, Novaldex, Novantrone, Paraplatin, Procrin, Prostacur, Suprefact, Tamoxifeno Funk, Taxol, Taxotere, Testex, Elmu/Prolongatum, Tomudex, Utefos, Vepesid, Xeloda, Zoladex;
(as active compounds): Anastrozole, Bicalutamide, Busereline, Capecetabine, Cisplatin, Carboplatin, Desoxorubicin, Docetaxel, Etoposid, Fulvestrant, Gemcitabine, Gosereline, Irinotecan, Letrozole, Leuproreline, Megestrol, Mitotane, Mitoxantrone, Oxaliplatin, Paclitaxel, Pemetrexed, Raltitrexed, Tamoxiphen, Tegafur, Triptoreline, Vincristine, Vinblastine, Vinorelbine, Vindesine.

Paclitaxel (Taxol®) is one of the most effective and commonly used antineoplastic drugs for the treatment of solid tumours. It has two serious side effects, myelosupression and peripheral neurotoxicity. Granulocyte colony-stimulating factor effectively counteracts the neutropenia in most patients. Unfortunately, there are no acceptable therapies to prevent or minimize the nerve damage, making neurotoxicity a significant dose-limiting side effect (Rowinsky et al., 1993a, b; Wasserheit et al., 1996; Gordon et al., 1997). Paclitaxel-induced neurotoxicity typically presents as a sensory neuropathy, with the most common complaints being numbness, tingling, burning pain and cold allodynia (Rowinsky et al., 1993a; Chaudhry et al., 1994; Forsyth et al., 1997; Dougherty et al., 2004). Sensory symptoms usually start symmetrically in the feet, but sometimes appear simultaneously in both hands and feet (Rowinsky et al., 1993a; Quasthoff and Hartung, 2002). A clinically significant number of patients with paclitaxel-induced neuropathy experience neuropathic pain. For example, in a study of 27 patients treated with paclitaxel doses of 135, 175 and 250-300 mg/m², neuropathic symptoms occurred in 50, 79 and 100% of patients, progressing to dose-limiting neurotoxicity in 0, 21 and 71% of patients, respectively (Postma et al., 1995).

"Neuropathic pain" is defined by the IASP as "pain initiated or caused by a primary lesion or dysfunction in the nervous system" (IASP, Classification of chronic pain, 2nd Edition, IASP Press (2002), 210). For the purpose of this invention included under this heading or to be treated as synonymous is "Neurogenic Pain" which is defined by the IASP as "pain initiated or caused by a primary lesion, dysfunction or transitory perturbation in the peripheral or central nervous system". In regards to this invention the neuropathic pain treated according to this invention is restricted to the neuropathic pain resulting from chemotherapy, meaning being caused by the use of a chemotherapeutic drug in chemotherapy. The most likely cause of this is neurotoxicity of the chemotherapeutic drug, especially peripheral neurotoxicity.

Neurotoxicity of the chemotherapeutic drug often leads to sensory neuropathy, which i.a. results in a burning pain and cold allodynia, but with also mechanical hyperalgesia and allodynia being seen as well as thermal (warm) hyperalgesia.

According to the IASP "allodynia" is defined as "a pain due to a stimulus which does not normally provoke pain" (IASP, Classification of chronic pain, 2nd Edition, IASP Press (2002), 210). According to the IASP "peripheral neuropathic pain" is defined as "a pain initiated or caused by a primary lesion or dysfunction in the peripheral nervous system" and "peripheral neurogenic pain" is defined as "a pain initiated or caused by a primary lesion, dysfunction or transitory perturbation in the peripheral nervous system" (IASP, Classification of chronic pain, 2nd Edition, IASP Press (2002), 213).

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

According to the IASP "hyperalgesia" is defined as "an increased response to a stimulus which is normally painful(IASP, Classification of chronic pain, 2nd Edition, IASP Press (2002), 211).

According to the IASP "hyperesthesia" is defined as "increased sensitivity to stimulation, excluding the senses" (IASP, Classification of chronic pain, 2nd Edition, IASP Press (2002), 211).

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold; Increased response | Stimulus and response rate may be the same or different |

According to the IASP "neuralgia" is defined as "Pain in the distribution of a nerve or nerves" (IASP, Classification of chronic pain, 2nd Edition, IASP Press (2002), 212).

According to the IASP "neuritis" is defined as "Inflammation of a nerve or nerves" (IASP, Classification of chronic pain, 2nd Edition, IASP Press (2002), 212).

According to the IASP "neuropathy/neuritis" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

"The sigma receptor/s" as used in this application is/are well known and defined using the following citation: This binding site represents a typical protein different from opioid, NMDA, dopaminergic, and other known neurotransmitter or hormone receptor families (G. Ronsisvalle et al. Pure Appl. Chem. 73, 1499-1509 (2001)). Pharmacological data based on ligand binding studies, anatomical distribution and biochemical features distinguish at least two subtypes of σ receptors (R. Quiron et al., Trends Pharmacol. Sci. 13, 85-86 (1992); M.L.Leitner, Eur. J. Pharmacol. 259, 65-69 (1994); S.B. Hellewell and W.D. Bowen; Brain Res. 527, 244-253 (1990)) (G. Ronsisvalle et al. Pure Appl. Chem. 73, 1499-1509 (2001)). The protein sequence of sigma receptors (Sigma 1 (σ1) and Sigma 2 (σ2)) is known (e.g. Prasad, P.D. et al., J. Neurochem. 70 (2), 443-451 (1998)) and they show a very high affinity for e.g. pentazocine.

"Compound/s binding to the sigma receptor" or "sigma ligand" as used in this application is/are defined as a compound having an IC₅₀ value of ≤5000 nM, more preferably ≤1000 nM, more preferably ≤500 nM on the sigma receptor. More preferably, the IC₅₀ value is ≤50 nM. More preferably, the IC₅₀ value is ≤100 nM. Most preferably, the IC₅₀ value is ≤ 50 nM. Additionally, the wording "Compound/s binding to the sigma receptor", as used in the present application is defined as having at least ≥50% displacement using 10 mM radioligand specific for the sigma receptor (e.g. preferably ¹H-pentazocine) whereby the sigma recepor may be any sigma receptor subtype. Preferably, said compounds bind to the sigma-1 receptor subtype.

Compounds binding to the sigma receptor - generally also known as sigma ligands - are well known in the art with many of them falling under the definition for "Compound/s binding to the sigma receptor" set up above. Still even though there are many uses known for sigma ligands such as antipsychotic drugs, anxiolytics, antidepressants, the treatment of stroke, antiepileptic drugs and many other indications including anti-migraine and general pain there is no mentioning of these compounds being useful against the symptoms of pain developing as a consequence of Chemotherapy.

Compounds binding to the sigma receptor known in the art and matching the criteria of sigma ligand (i.e. having an IC₅₀ ≤ 5000 nM) as mentioned above, are listed below. Some of these compounds may bind to the sigma-1 and/or the sigma-2 receptor. Preferably, these compounds are in form of a salt, a base or an acid. Also preferably, the salts/bases/acids indicated in the list are to be understood as being exemplary and therefore may respresent any salt, base or acid of the compound.

| | |
|---|---|
| (-)-Cyanopindolol hemifumarate | (-)-SPARTEINE SULFATE PENTAHYDRATE |
| (+)-HIMBACINE | (2-Dibutylamino-Ethyl)-Carbamic Acid 2-(4-Benzofuran-2-Ylmethyl-Piperazin-1-YI)-Ethyl Ester |
| (4-[1,2,3]Thiadiazol-4-Yl-Benzyl)-Carbamic Acid 1-(3-Methoxy-2-Nitro-Benzyl)-Piperidin-3-Ylmethyl Ester | (S)-Methamphetamine HCl |
| [1-(9-Ethyl-9H-Carbazol-3-Ylmethyl)-Pyrrolidin-3-Yl]-Carbamic Acid 1-(3-Benzyloxy-4-Methoxy-Benzyl)-Piperidin-3-Ylmethyl Ester | [1-(9-Ethyl-9H-Carbazol-3-Ylmethyl)-Pyrrolidin-3-Yl]-Carbamic Acid 2-(Tert-Butoxycarbonyl-Naphthalen-1-Ylmethyl-Amino)-Ethyl Ester |
| [4-(4-Ethyl-3,5-Dimethyl-Pyrazol-1-Yl)-Phenyl]-[4-(3-Phenyl-Allyl)-Piperazin-1-Yl]-Methanone | 1-(1,2-Diphenylethyl)Piperidine Maleate, (+/-) |
| 1-(1-Naphthyl)Piperazine HCl | 1-(3-Chlorophenyl)Piperazine HCl |
| 1-(4-Bromo-Benzenesulfonyl)-4-(2-Tert-Butylsulfanyl-Benzyl)-Piperazine | 2-(2-{[1-(3-Ch)oro-Benzy))-Pyrrolidin-3-Yl]-Methyl-Carbamoyl)-2-Methyl-Propyl)-4,6-Dimethyl-Benzoic Acid |
| 2-Chloro-11-(4-Methylpiperazino)Dibenz[B,F]Oxepin Maleate | 3,3'-Diethylthiacarbocyanine lodide |
| 3-Mercapto-2-Methylpropanoic Acid 1,2-Diphenylethylamine Salt | 3-Quinuclidinyl Benzilate |
| 3-Tropanyl-3,5-Dichlorobenzoate | 3-Tropanyl-Indole-3-Carboxylate HCl |
| 4-(1H-lndol-4-Yl)-Piperazine-1-Carboxylic Acid 2-(5-Bromo-2-Ethoxy-Phenylamino)-Cyclohexylmethyl Ester | 4-(2-Tert-Butylsulfanyl-Benzyl)-Piperazine-1-Carboxylic Acid 2-Thiophen-2-Yl-Ethyl Ester |
| 4-(3,5-Dimethoxy-Phenyl)-Piperazine-1- | 4-(3-Nitro-5-Sulfamoyl-Thiophen-2-Yl)- |
| Carboxylic Acid 1-(2-Fluoro-Benzyl)-Piperidin-2-Ylmethyl Ester | Piperazine-1-Carboxylic Acid 1-(2-Fluoro-5-Methoxy-Benzyl)-Piperidin-3-Ylmethyl Ester |
| 4-(4-Fluorobenzoyl)-1-(4-Phenylbutyl)Piperidine Oxalate | 4-(5-Trifluoromethyl-Pyridin-2-Yl)-Piperazine-1-Carboxylic Acid Pent-2-Ynyl Ester |
| 4,4'-Bis[4-(P-Chlorophenyl)-4-Hydroxypiperidino]Butyrophenone | 4-[1-(4-Chlorobenzyl)-4-(benzylpiperidin-4-yl]-2-hydroxy-4-oxobut-2-enoic acid |
| 4-Bromo-N-[1-(9-Ethyl-9H-Carbazol-3-Ylmethyl)-Pyrrolidin-3-Yl]-2-Trifluoromethoxy-Benzenesulfonamide | 4'-Chloro-3-Alpha-(Diphenylmethoxy)Tropane HCl |
| 4-Furan-2-Ylmethyl-Piperazine-1-Carboxylic Acid 2-{4-[3-(2-Trifluoromethyl-Phenothiazin-10-Yl)-Propyl]-Piperazin-1-Yl)-Ethyl Ester | 4-Methoxy-N-[1-(7-Methoxy-Benzo[1,3]Dioxol-5-Ylmethyl)-Pyrrolidin-3-Yl]-Benzenesulfonamide |
| 5-(N-Ethyl-N-isopropyl)-Amiloride | 7-Hydroxy-DPAT HBr, (±)- |
| 8-Hydroxy-DPAT HBr, (R)-(+)- | 8-Hydroxy-DPAT HBr, S(-)- |
| 9-[4-({[4'-(trifluoromethyl)-1,1'-biphenyl-2-yl]carbonyl}amino)piperidin-1-yl]-N-(2,2,2-trifluoroethyl)-9H-fluorene-9-carboxamide | Acepromazine Maleate |
| Acetophenazine Maleate | Acrinol |
| Ajmaline | Alaproclate HCl |
| Aloe-Emodin | Alprenolol D-Tartrate Salt Hydrate |
| Alprenolol HCl | AMI-193 |
| Aminobenztropine | Amiodarone HCl |
| Amodiaquine HCl | Amorolfine HCl |
| Amoxapine | Anileridine HCl |
| Anisotropine Methylbromide | Anpirtoline |
| ARC 239 DiHCl | Astemizole |
| Auramine O HCl | Azaperone |
| Azatadine Maleate | Azelastine HCl |
| Bamethan sulfate | BD 1008 DiHBr |
| BD-1047 | BD-1063 |
| Benextramine TetraHCl | Benfluorex HCl |
| Benidipine HCl | Benoxathian HCl |
| Benoxinate HCl | Benperidol |
| Benproperine Phosphate | Benzododecinium bromide |
| Benzphetamine HCl | Benztropine Mesylate |
| Benzydamine HCl | Bephenium Hydroxynaphthoate |
| Bepridil HCl | Berberine chloride |
| Betaxolol HCl | Bifemelane |
| BMY 7378 DiHCl | Bopindolol Malonate |
| BP 554 Maleate | Bromhexine HCl |
| Bromodiphenhydramine HCl | Bromperidol |
| Brompheniramine Maleate | BTCP HCl |
| Buclizine HCl | Buflomedil HCl |
| Bupropion HCl | Buspirone HCl |
| Butacaine Sulfate | Butaclamol HCl, (±)- |
| Butenafine HCl | Butoconazole Nitrate |
| BW 723C86 HCl | Carbetapentane Citrate |
| Carbinoxamine Maleate | Carpipramine DiHCl DiH2O |
| Carvedilol | Cephapirin Benzathine |
| CGS-12066A Maleate | Chloroprocaine HCl |
| Chloroquine Phosphate | Chlorpheniramine Maleate |
| Chlorphenoxamine HCl | Chlorpromazine HCl |
| Chlorprothixene | Cinanserin HCl |
| Cinnarizine | Cirazoline HCl |
| Cis-(+/-)-N-Methyl-N-[2-(3,4-Dichlorophenyl)Ethyl]-2-(1-Pyrrolidinyl)Cyclohexamine DiHBr | Cis(Z)-Flupentixol DiHCl |
| Cisapride Hydrate | Citalopram HBr |
| Clebopride Maleate Salt | Clemastine Fumarate |
| Clemizole HCl | Clenbuterol HCl |
| Clidinium Bromide | Clobenpropit 2HBr |
| Clofazimine | Clofilium Tosylate |
| Clomiphene Citrate | Clomiphene Related Compound A |
| Clomipramine | Cloperastine HCl |
| Clorgyline HCl | Clozapine |
| CONESSINE | Cyclizine |
| Cyclobenzaprine HCl | Cycloheximide |
| Cyproheptadine HCl | Darrow Red HCl |
| Demecarium Bromide | Denatonium Benzoate |
| Deptropine Citrate | Desloratadine |
| Dexbrompheniramine Maleate | Dexchlorpheniramine Maleate |
| Dexfenfluramine HCl | Dibucaine HCl |
| Dicyclomine HCl | Diethylpropion HCl |
| Dimethisoquin HCl | Dimetindene Maleate |
| Diphemanil Methylsulfate | Diphenidol HCl |
| Diphenoxylate HCl | Diphenylpyraline HCl |
| Dipropyldopamine HBr | Dobutamine HCl |
| Donepezil HCl | Doxepin HCl |
| Droperidol | Duloxetine |
| Dyclonine HCl | Ebastine |
| Econazole Nitrate | Epinastine HCl |
| Ethaverine HCl | Ethopropazine HCl |
| Eticlopride HCl, S(-)- | Etofenamate |
| Etonitazenyl Isothiocyanate | Femoxetine HCl |
| Fenfluramine HCl | Fentanyl Citrate |
| Fenticonazole Nitrate | Fipexide HCl |
| Flavoxate HCl | Flunarizine diHCl |
| Fluoxetine Related Compound B | Fluperlapine |
| Fluphenazine Decanoate DiHCl | Fluphenazine Enanthate DiHCl |
| Fluphenazine HCl | Fluphenazine N-Mustard DiHCl |
| Flurazepam Related Compound C | Fluspirilene |
| Fluvoxamine Maleate | GBR 12783 DiHCl |
| GBR 12909 DiHCl | GBR 13069 DiHCl |
| GBR-12935 DiHCl | GR 89696 Fumarate |
| Guanabenz Acetate | Guanadrel Sulfate |
| Guanethidine Sulfate | Halofantrine HCl |
| Haloperidol | HEAT HCl |
| Hexylcaine HCl | Hycanthone |
| Hydroxychloroquine Sulfate | Hydroxyzine HCl |
| Hyoscyamine Sulfate | IBZM, S(-)- |
| ICI-199,441 HCl | Ifenprodil Tartrate |
| Imipramine HCl | Indatraline HCl |
| lofetamine HCl | Irinotecan HCl |
| Isamoltane Hemifumarate | Isopromethazine HCl |
| Isoxsuprine HCl | Ketanserin L-Tartrate |
| Ketoconazole | Ketotifen Fumarate Salt |
| L-693,403 Maleate | L-741,626 |
| L-741,742 HCl | L-745,870 TriHCl |
| Labetalol HCl | Levetimide HCl, R(-) |
| Levobunolol HCl | Lidoflazine |
| Lisuride Hydrogen Maleate, R(+)- | Lobeline HCl |
| lomerizine diHCl | Loperamide HCl |
| Loxapine Succinate | LY-53,857 Maleate |
| Maprotiline HCl | Mazindol |
| MDL 12,330A HCl | Mebhydroline 1,5-naphthalendisulfonate Salt |
| Meclizine HCl | Mefloquine HCl |
| Meprylcaine HCl | Mesoridazine Besylate |
| Metaphit Methanesulfonate | Metergoline |
| Methantheline Bromide | Methdilazine |
| Methiothepin Mesylate | Methixene HCl |
| Methoctramine | Methotrimeprazine Maleate |
| Methylene Violet 3Rax HCl | Metipranolol |
| Mexiletine HCl | Mianserin HCl |
| Miconazole | ML-9 HCl |
| Morantel Hydrogen L-Tartrate | MR 16728 HCl |
| N-(2-Chloroethyl)-N-Ethyl-2-Bromobenzylamine HCl | N'-[2-(Benzo[1,2,5]Thiadiazole-4-Sulfonylamino)-Acetyl]-Hydrazinecarboxylic Acid 2-(2-{4-[(4-Chloro-Phenyl)-Phenyl-Methyl]-Piperazin-1-Yl}-Ethoxy)-Ethyl Ester |
| Nafronyl Oxalate Salt | Naftifine |
| Naftopidil diHCl | Naltriben Mesylate |
| NAN-190 HBr | NE-100 |
| Nefazodone | Nefopam HCl |
| Nicardipine HCl | Nicergoline |
| Niguldipine HCl, (+/-)- | Nisoxetine HCl |
| Nortriptyline HCl | Nylidrin HCl |
| Octoclothepin Maleate, (±)- | Orphenadrine Citrate |
| Oxamniquine | Oxamniquine Related Compound A |
| Oxamniquine Related Compound B | Oxatomide |
| Oxiconazole Nitrate | Oxybutynin HCl |
| Panaxatriol | PAPP |
| Paroxetine | Paxilline |
| p-Chlorobenzhydrylpiperazine | Penbutolol Sulfate |
| Pentamidine Isethionate | Pentazocine, (±)- |
| Pergolide Methanesulfonate | Perhexiline Maleate Salt |
| Perospirone | Perphenazine |
| Perphenazine Sulfoxide | Phenamil Methanesulfonate |
| Phencyclidine HCl | Phenosafranin HCl |
| Phenoxybenzamine HCl | Phenyltoloxamine Citrate Salt |
| Piboserod | Pimozide |
| Pinacyanol Chloride | Pindobind, (+/-)- |
| Piperacetazine | Piperazine-1,4-Dicarboxylic Acid Benzyl Ester 2-[4-(4-Dimethylamino-Benzyl)-Piperazin-1-Yl]-Ethyl Ester |
| Piperidolate HCl | Pirenperone |
| PPHT HCl, (±)- | Pramoxine HCl |
| Prenylamine Lactate Salt | Pridinol Methanesulfonate Salt |
| Prochlorperazine Maleate | Procyclidine HCl |
| Proflavine Hemisulfate Salt | Progesterone |
| Promazine HCl | Promethazine HCl |
| Propafenone HCl | Proparacaine HCl |
| Propericyazine | Propiomazine |
| Propranolol HCl | Protokylol |
| Protriptyline HCl | Pyrilamine Maleate |
| Pyrimethamine | Pyrrolidine-1,2-Dicarboxylic Acid 1-[1-(4-Allyloxy-Benzyl)-Piperidin-2- Ylmethyl] Ester 2-Benzyl Ester |
| Pyrvinium Pamoate | Quetiapine Fumarate |
| Quinacrine HCl | Quinaldine Red |
| Quipazine Dimaleate | Quipazine, 6-Nitro-, Maleate |
| Raloxifene | Rimantadine HCl |
| Risperidone | Ritanserin |
| Ritodrine HCl | RS 23597-190 HCl |
| RS 67333 HCl | RS 67506 HCl |
| Safranin O HCl | Salmeterol |
| SB203186 | SCH-23390 HCl, R(+)- |
| Sertaconazole Nitrate | Sertindole |
| Sertraline | Sibutramine HCl |
| SKF-525A HCl | SKF-96365 HCl |
| SNC 121 | Spiperone HCl |
| Sufentanil | T-226296 |
| Tamoxifen Citrate | Tamsulosin HCl |
| Tegaserod Maleate | Terbinafine HCl |
| Terconazole | Terfenadine |
| Terfenadine Related Compound A | Tetracaine HCl |
| Tetrindole Mesylate | Thiethylperazine Malate |
| Thioperamide Maleate | Thioproperazine |
| Thioridazine | Thiothixene |
| Thiothixene, (E)- | Thonzonium Bromide |
| Tioconazole Related Compound A | TMB-8 HCl |
| Tolterodine L-Tartrate | Toremifene Citrate |
| Tramazoline HCl | Trans-U-50488 Methanesulfonate, (±)- |
| Trazodone HCl | Tridihexethyl Chloride |
| Trifluoperazine HCl | Trifluperidol HCl |
| Triflupromazine HCl | Trihexyphenidyl HCl |
| Trimebutine | Trimeprazine Hemi-L-Tartrate |
| Trimipramine Maleate | Tripelennamine HCl |
| Triprolidine HCl | Triprolidine HCl Z Isomer |
| Tropanyl 3,5-Dimethylbenzoate | Tropine 2-(4-Chlorophenoxy)Butanoate, Maleate |
| U-50488 HCl, (-)- | U-62066 |
| UH 232 Maleate, (+)- | Vecuronium Bromide |
| Verapamil HCl | Verapamil Related Compound B |
| Vesamicol HCl | Vinpocetine |
| W-7 HCl | WB-4101 HCl |
| Xylazine | Xylometazoline HCl |

In the following additional list of compounds binding to the sigma receptor known in the art and matching the criteria of sigma ligand, but with a higher affinity (i.e. having an IC₅₀ ≤ 250 nM), are listed below. Some of these compounds may bind to the sigma-1 and/or the sigma-2 receptor. Preferably, these compounds are in form of a salt, a base or an acid. Also preferably, the salts/bases/acids indicated in the list are to be understood as being exemplary and therefore may respresent any salt, base or acid of the compound.

| | |
|---|---|
| (2-Dibutylamino-Ethyl)-Carbamic Acid 2-(4-Benzofuran-2-Ylmethyl-Piperazin-1-Yl)-Ethyl Ester | (4-[1,2,3]Thiadiazol-4-Yl-Benzyl)-Carbamic Acid 1-(3-Methoxy-2-Nitro-Benzyl)-Piperidin-3-Ylmethyl Ester |
| 4-(4-Fluorobenzoyl)-1-(4-Phenylbutyl)Piperidine Oxalate | 4-[1-(4-Chlorobenzyl)-4-(benzylpiperidin-4-yl]-2-hydroxy-4-oxobut-2-enoic acid |
| 4-Bromo-N-[1-(9-Ethyl-9H-Carbazol-3-Ylmethyl)-Pyrrolidin-3-Yl]-2-Trifluoromethoxy-Benzenesulfonamide | 4'-Chloro-3-Alpha-(Diphenylmethoxy)Tropane HCl |
| 4-Furan-2-Ylmethyl-Piperazine-1-Carboxylic Acid 2-{4-[3-(2-Trifluoromethyl-Phenothiazin-10-Yl)-Propyl]-Piperazin-1-Yl}-Ethyl Ester | Acetophenazine Maleate |
| Aminobenztropine | Amiodarone HCl |
| Amodiaquine HCl | Amorolfine HCl |
| Anileridine HCl | Astemizole |
| Azaperone | Azelastine HCl |
| BD 1008 DiHBr | BD-1047 |
| BD-1063 | Benextramine TetraHCl |
| Benfluorex HCl | Benoxathian HCl |
| Benperidol | Benproperine Phosphate |
| Benzododecinium bromide | Benztropine Mesylate |
| Bepridil HCl | Berberine chloride |
| Bifemelane | BP 554 Maleate |
| Bromhexine HCl | Bromodiphenhydramine HCl |
| Bromperidol | Buflomedil HCl |
| Butacaine Sulfate | Butaclamol HCl, (±)- |
| Butenafine HCl | Carbetapentane Citrate |
| Carpipramine DiHCl DiH20 | Cinnarizine |
| Cis-(+/-)-N-Methyl-N-[2-(3,4-Dichlorophenyl)Ethyl]-2-(1-Pyrrolidinyl)Cyclohexamine DiHBr | Cis(Z)-Flupentixol DiHCl |
| Cisapride Hydrate | Clofilium Tosylate |
| Clomiphene Citrate | Clomiphene Related Compound A |
| Clomipramine | Cloperastine HCl |
| Clorgyline HCl | Cyclobenzaprine HCl |
| Cyproheptadine HCl | Demecarium Bromide |
| Deptropine Citrate | Dibucaine HCl |
| Dicyclomine HCl | Diphenylpyraline HCl |
| Donepezil HCl | Doxepin HCl |
| Dyclonine HCl | Femoxetine HCl |
| Flunarizine diHCl | Fluphenazine Decanoate DiHCl |
| Fluphenazine Enanthate DiHCl | Fluphenazine HCl |
| Fluphenazine N-Mustard DiHCl | GBR 12783 DiHCl |
| GBR 12909 DiHCl | GBR 13069 DiHCl |
| GBR-12935 DiHCl | Haloperidol |
| HEAT HCl | Hexylcaine HCl |
| Hydroxyzine HCl | Ifenprodil Tartrate |
| Isopromethazine HCl | Isoxsuprine HCl |
| L-693,403 Maleate | L-741,626 |
| L-741,742 HCl | L-745,870 TriHCl |
| Lidoflazine | Lobeline HCl |
| lomerizine diHCl | Loperamide HCl |
| LY-53,857 Maleate | Metergoline |
| Methdilazine | Methixene HCl |
| Metipranolol | ML-9 HCl |
| MR 16728 HCl | Naftifine |
| Naftopidil diHCl | NAN-190 HBr |
| Nicardipine HCl | Nylidrin HCl |
| Octoclothepin Maleate, (±)- | Oxamniquine Related Compound A |
| Oxybutynin HCl | PAPP |
| Penbutolol Sulfate | Pentazocine, (±)- |
| Perphenazine | Phenoxybenzamine HCl |
| Pimozide | Piperidolate HCl |
| PPHT HCl, (±)- | Prenylamine Lactate Salt |
| Prochlorperazine Maleate | Promazine HCl |
| Proparacaine HCl | Protriptyline HCl |
| Pyrrolidine-1,2-Dicarboxylic Acid 1-[1-(4-Allyloxy-Benzyl)-Piperidin-2-Ylmethyl] Ester 2-Benzyl Ester | Pyrvinium Pamoate |
| Raloxifene | Ritanserin |
| RS 67333 HCl | RS 67506 HCl |
| Salmeterol | Sertindole |
| Sertraline | SKF-525° HCl |
| Tamoxifen Citrate | Tegaserod Maleate |
| Terbinafine HCl | Terconazole |
| Thioridazine | Toremifene Citrate |
| TMB-8 HCl | Trifluperidol HCl |
| Trifluoperazine HCl | Trimeprazine Hemi-L-Tartrate |
| Triflupromazine HCl | Tripelennamine HCl |
| Trimipramine Maleate | Verapamil HCl |
| U-50488 HCl, (-)- | Xylazine |
| WB-4101 HCl | |

Examples of well known methods of producing a prodrug of a given acting compound are known to those skilled in the art and can be found e.g. in Krogsgaard-Larsen et al., Textbook of Drugdesign and Discovery, Taylor & Francis (April 2002).

In this application "about" means "approximately," and illustratively, the use of the term "about" indicates that dosages slightly outside the cited ranges may also be effective and safe, and such dosages are also encompassed by the scope of the present claims.

Compounds that are "administered together with compounds binding to the sigma receptor" or "in combination with compounds binding to the Sigma receptor" may be administered as part of the same composition, or may be administered separately, at the same or at separate times, in the same therapeutic regimen.

In connection with this invention "neutral form" refers either to a non-ionic form or to a neutrally net charged form, for example a Zwitter-lon at its isoelectric point.

The term "salt" according to this invention is to be understood as meaning any form of the active compound according to the invention in which this compound assumes an ionic form or is charged and - if applicable - is also coupled with a counter-ion (a cation or anion). By this are also to be understood complexes of the active compound with other molecules and ions, in particular complexes which are complexed via ionic interactions. As preferred examples of salts this includes the acetate, mono-trifluoracetate, acetate ester salt, citrate, formate, picrate, hydrobromide, monohydrobromide, monohydrochloride or hydrochloride.

The term "physiologically acceptable salt" in the context of this invention is understood as meaning a "salt" (as defined above) of at least one of the compounds according to the invention which are physiologically tolerated by humans and/or mammals.

The term "solvate" according to this invention is to be understood as meaning any form of the active compound according to the invention which has another molecule (most likely a polar solvent) attached to it via non-covalent bonding. Examples of solvates include hydrates and alcoholates, e.g. methanolate.

The term "treatment" or "to treat" in the context of this specification means administration of a compound or formulation according to the invention to prevent, ameliorate or eliminate one or more symptoms associated with neuropathic pain, hyperalgesia and/or allodynia.

Furthermore, the terms "to treat" or "treatment" according to this invention include the treatment of symptoms of neuropathic pain, hyperalgesia and/or allodynia, the prevention or the prophylaxis of the symptoms of neuropathic pain, hyperalgesia and/or allodynia, the prevention or prophylaxis causing the symptoms of neuropathic pain, hyperalgesia and/or allodynia, as well as the prevention or the prophylaxis of the consequences causing the symptoms.

According to the various embodiments, the compounds binding to the sigma receptor or the pharmaceutical compositions comprising them, may be administered, in unit dosage form, intestinally, enterally, parenterally or topically, orally, subcutaneously, intranasally, by inhalation, by oral absorption, intravenously, intramuscularly, percutaneously, intraperitoneally, rectally, intravaginally, transdermally, sublingually, buccally, orally transmucosally. Administrative dosage forms may include the following: tablets, capsules, dragees, lozenges, patches, pastilles, gels, pastes, drops, aerosols, pills, powders, liquors, suspensions, emulsions, granules, ointments, creams, suppositories, freeze-dried injections, injectable compositions, in food supplements, nutritional and food bars, syrups, drinks, liquids, cordials etc, which could be regular preparation, delayed-released preparation, controlled-released preparation and various micro-granule delivery system, in food supplements, nutritional and food bars, syrups, drinks, liquids, cordials. In case of tablet, various carriers known in the art may be used, e.g. dilutent and resorbent such as starch, dextrin, calcium sulfate, kaolin, microcrystalline cellulose, aluminium silicate, etc; wetting agent and adhesives such as water, glycerin, polyethylene glycol, ethanol, propanol, starch mucilage, dextrin, syrup, honey, glucose solution, acacia, gelatin, carboxymethylcellulose sodium, shellac, methylcellulose, potassium phosphate, polyvinylpyrrolidone, etc; disintegrating agent, such as dried starch, alginate, agar powder, laminaran, sodium bicarbonate and citric acid, calcium carbonate, polyoxyethylene sorbitol aliphatic ester, lauryl sodium sulfate, methylcellulose, ethylcellulose, lactose, sucrose, maltose, mannitol, fructose, various disaccharides and polysaccharides etc; disintegration inhibiting agent, such as sucrose, tristearin, cacao butter, hydrogenated oil, etc; absorption accelerator, such as quaternary ammonium salt, lauryl sodium sulfate, etc; lubricant, such as talc, silica, corn starch, stearate, boric acid, fluid wax, polyethylene, etc. The tablet may be further formulated into coated tablet, e.g. sugar-coated tablet, film-coated tablet, enteric-coated tablet, or double-layer tablet and multi-layer tablet. In the case of pill, various carriers known in the art may be used, e.g. dilutent and resorbent, such as glucose, lactose, starch, cacao butter, hydrogenated vegetable oil, polyvinylpyrrolidone, kaolin, talc, etc; adhesives, such as acacia, bassora gum, gelatin, ethanol, honey, liquid sugar, rice paste or flour paste, etc; disintegrating agent, such as agar powder, dried starch, alginate, lauryl sodium sulfate, methylcellulose, ethylcellulose. In case of suppository, various carriers known in the art may be used, e.g. polyethylene, lecithin, cacao butter, higher alcohols, esters of higher alcohols, gelatin, semi-synthetic glyceride, etc. In the case of capsule, it may be prepared by mixing said sodium channel blockers as active ingredient with the above mentioned carriers, followed by placing the mixture into a hard gelatin capsule or soft capsule. Also, said sodium channel blockers may be applied in the following dosage forms: microcapsules, suspension in an aqueous phase, hard capsule, or injection. In the case of injection, such as liquor, emulsion, freeze-dried injection, and suspension, all the dilutents common in the art may be used, e.g. water, ethanol, polyethylene glycol, propylene glycol, oxyethylated isostearyl alcohol, polyoxidated isostearyl alcohol, polyoxyethylene sorbitol aliphatic ester, etc. In addition, in order to obtain isotonic injection, a suitable amount of sodium chloride, glucose or glycerin may be added into the preparation, as well as regular cosolvent, buffer, pH adjusting agent, etc. In addition, coloring agent, antiseptic, perfume, correctives, food sweetening agent or other materials may be added to the pharmaceutical preparation if necessary.

In certain embodiments a formulation or pharmaceutical composition according to the invention contains the active ingredient (a compound binding to the sigma receptor) as well as optionally at least one auxiliary material and/or additive and/or optionally another active ingredient.

In certain embodiments the auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

In certain embodiments routes of administration of the compound binding to the sigma receptor can include intramuscular injection, intraveneous injection, subcutaneous injection, sublingual, bucal, patch through skin, oral ingestion, implantable osmotic pump, collagen implants, aerosols or suppository.

In alternative embodiments the compounds binding to the sigma receptor may be administered in a schedule of one, two, three, four, five, six, seven, eight, nine, ten, eleven, twelve, thirteen, fourteen, fifteen, sixteen, seventeen, eighteen, nineteen, twenty or more doses per day, alone or in combination with other medications, over range of time periods including but not limited to periods of one, two, three, four, five, six, seven, eight, nine, ten, eleven, twelve, thirteen, fourteen, sixteen, eighteen, twenty, twenty four, thirty, or more days; or over a period of one, two, three, four, five, six, seven, eight, nine, ten, eleven, twelve, thirteen, fourteen, sixteen, eighteen, twenty, twenty four, thirty, thirty six, forty eight, sixty, seventy two, eighty four or more months.

In some embodiments the effectiveness of a course of treatment of one, two, three, four, five or more doses or one, two or three days may last for up to about five, ten, fifteen, twenty, twenty five or thirty. In some embodiments dosing is only performed once every day or once every two, three, four, five, six, seven, eight, nine, ten, eleven, twelve, thirteen, fourteen, sixteen, eighteen, twenty, twenty four, thirty or more days.

According to the present disclosure, the dosage of the compound binding to the sigma receptor depends on a variety of factors, including the nature and severity of the diseases, the sex, age, weight and individual reaction of the subject, the particular compound employed, the route and frequency of administration, etc. Said compounds binding to the sigma receptor or the pharmaceutical compositions comprising them may be administered in single or divided dosage form, e.g. one to four doses per day. Those skilled in the art will readily understand and implement the changes to the treatment methods exemplified herein that are necessary or desirable to reflect varied therapeutic requirements.

In a highly preferred embodiment of the use according to the invention the neuropathic pain is peripheral neuropathic pain or peripheral neurogenic pain.

According to the IASP "peripheral neuropathic pain" is defined as "a pain initiated or caused by a primary lesion or dysfunction in the peripheral nervous system" and "peripheral neurogenic pain" is defined as "a pain initiated or caused by a primary lesion, dysfunction or transitory perturbation in the peripheral nervous system" (IASP, Classification of chronic pain, 2nd Edition, IASP Press (2002), 213).

In another preferred embodiment of the use according to the invention the neuropathic pain is allodynia.

According to the IASP "allodynia" is defined as "a pain due to a stimulus which does not normally provoke pain" (IASP, Classification of chronic pain, 2nd Edition, IASP Press (2002), 210).

In another preferred embodiment of the use according to the invention the neuropathic pain is causalgia.

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

In another preferred embodiment of the use according to the invention the neuropathic pain is hyperalgesia.

According to the IASP "hyperalgesia" is defined as "an increased response to a stimulus which is normally painful(IASP, Classification of chronic pain, 2nd Edition, IASP Press (2002), 211).

In another preferred embodiment of the use according to the invention the neuropathic pain is hyperesthesia.

According to the IASP "hyperesthesia" is defined as "increased sensitivity to stimulation, excluding the senses" (IASP, Classification of chronic pain, 2nd Edition, IASP Press (2002), 211).

In another preferred embodiment of the use according to the invention the neuropathic pain is hyperpathia.

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold; Increased response | Stimulus and response rate may be the same or different |

In another preferred embodiment of the use according to the invention the neuropathic pain is neuralgia.

According to the IASP "neuralgia" is defined as "Pain in the distribution of a nerve or nerves" (IASP, Classification of chronic pain, 2nd Edition, IASP Press (2002), 212).

In another preferred embodiment of the use according to the invention the neuropathic pain is neuritis.

According to the IASP "neuritis" is defined as "Inflammation of a nerve or nerves" (IASP, Classification of chronic pain, 2nd Edition, IASP Press (2002), 212).

In another preferred embodiment of the use according to the invention the neuropathic pain is neuropathy/neuritis.

According to the IASP "neuritis" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

In another preferred embodiment of the use according to the invention the neuropathic pain is orofacial pain.

Another aspect of the present invention relates to the use of a compound binding to the sigma receptor, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in form of a salt, especially a physiologically acceptable salt, or in form of a solvate, especially a hydrate for the treatment of allodynia developing as a consequence of Chemotherapy.

Another aspect of the present invention relates to the use of a compound binding to the sigma receptor, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in form of a salt, especially a physiologically acceptable salt, or in form of a solvate, especially a hydrate for the production of a medicament for the treatment of hyperalgesia developing as a consequence of chemotherapy.

In a highly preferred embodiment of the invention at least one chemotherapeutic agent used in the chemotherapy is selected from a platin-derivative, a vinca alkaloid or a taxane.

In a highly preferred embodiment of the invention at least one chemotherapeutic agent used in the chemotherapy is selected from the group consisting of cisplatin, carboplatin and oxaliplatin; vincristine, vinblastine, vinorelbine and vindesine; paclitaxel and docetaxel.

Preferably at least one chemotherapeutic agent used in the chemotherapy is cisplatin.

Preferably at least one chemotherapeutic agent used in the chemotherapy is carboplatin.

Preferably at least one chemotherapeutic agent used in the chemotherapy is oxaliplatin.

Preferably at least one chemotherapeutic agent used in the chemotherapy is vincristine.

Preferably at least one chemotherapeutic agent used in the chemotherapy is vinblastine.

Preferably at least one chemotherapeutic agent used in the chemotherapy is vinorelbine.

Preferably at least one chemotherapeutic agent used in the chemotherapy is vindesine.

Preferably at least one chemotherapeutic agent used in the chemotherapy is paclitaxel.

Preferably at least one chemotherapeutic agent used in the chemotherapy is docetaxel.

Included in this invention are especially also methods of treatment of a patient or a mammal, including men, suffering from neuropathic pain developing as a consequence of Chemotherapy using a compound binding to the sigma receptor optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate. It is also prefer if the method of treatment is applying to embodiments in which the compound binding to the sigma receptor is selected from compounds ... It is also preferred if the method of treatment is applying to embodiments in which at least one chemotherapeutic agent used in the chemotherapy is selected from a platin-derivative, a vinca alkaloid or a taxane; especially if at least one chemotherapeutic agent used in the chemotherapy is selected from the group consisting of cisplatin, carboplatin and oxaliplatin; vincristine, vinblastine, vinorelbine and vindesine; paclitaxel and docetaxel

Especially the above method of treatment includes also a co-treatment during chemotherapy, in which the chemotherapeutic drug/drugs are administered together with the compound binding to the sigma receptor or in combination with a compound binding to the sigma receptor. This co-treatment could possible happen during the whole chemotherapy, during parts of the chemotherapy, in the beginning of the chemotherapy, in the middle of the chemotherapy or at the end of the chemotherapy.

Another highly preferered embodiment is thus the use according to the invention for the treatment or prevention of pain developing as a consequence of chemotherapy, wherein the compound binding to the sigma receptor is combined with a a least one chemotherapeutic drug (B) forming a fixed-dose active substance combination. Desirably the chemotherapeutic drug (B) in this active substance combination is selected from a platin-derivative, a vinca alkaloid or a taxane, especially the chemotherapeutic drug (B) is selected from is selected from cisplatin, carboplatin and oxaliplatin; vincristine, vinblastine, vinorelbine and vindesine; paclitaxel and docetaxel.

Another alternative embodiment of the present invention refers to a kit comprising a compound binding to the sigma receptor, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate.

The orofacial region, the face and mouth, represent sites of some of the most common pains in the body. Epidemiological studies have revealed the high prevalence of several orofacial pain conditions such as temporomandibular disorders (TMD), burning mouth syndrome, and toothaches, (Dworkin, 2001; Feinman and Newton-John, 2004; LeResche, 2001; Lipton et al., 2001). Many of the difficulties experienced by clinicians with the management of acute and chronic orofacial pain conditions stem from a lack of recognition and understanding of their complex factors and interactions, from uncertainties of the aetiology or pathogenesis of many of the conditions, as well as from the lack of information of the comparative effectiveness of the analgesic drugs on orofacial pain.

Thus, a preferred aspect of the invention is the use of a compound binding to the sigma receptor, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in form of a salt, especially a physiologically acceptable salt, or in form of a solvate, especially a hydrate for the treatment of orofacial pain, preferably in the form of neuropathic pain, hyperalgesia or allodynia, more preferably in the form of neuropathic pain, hyperalgesia or allodynia, developing as a consequence of chemotherapy.

The examples and figures in the following section describing pharmacological trials are merely illustrative and the invention cannot be considered in any way as being restricted to these applications.

### Examples

### Pharmacological Experiments

Recently, models of paclitaxel-induced painful neuropathy have been developed in mice and rats. These models demonstrated that repeated administration of paclitaxel produced mechanical hyperalgesia and allodynia (Authier et al., 2000; Polomano et al., 2001; Dina et al., 2001 y 2004; Smith et al., 2004; Flatters y Bennett, 2004), cold allodynia (Polomano et al., 2001; Smith et al., 2004; Flatters y Bennett, 2004) and in some studies a thermal (warm) hyperalgesia (Polomano et al., 2001; Dina et al., 2001; Flatters y Bennett, 2004); however, other studies did not find this thermal hyperalgesia (Authier et al., 2000; Smith et al., 2004). Nevertheless, paclitaxel-induced painful neuropathy in rodents represents an interesting model to test the effects of drugs in neuropathic pain.

### FIGURES:

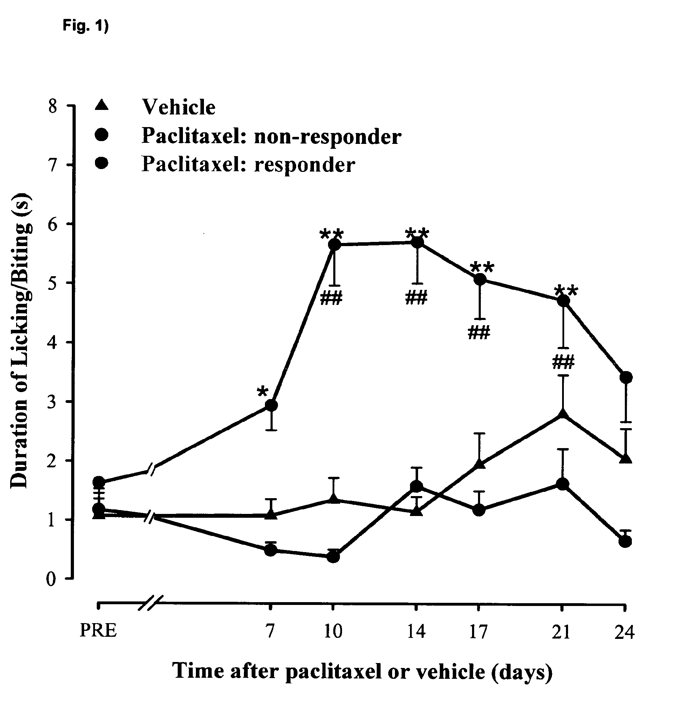

**Fig. 1**: Time-course of paclitaxel induced cold-allodynia in mice. Animals were treated once daily from days 1 to 5 with paclitaxel (2 mg/kg) or its vehicle via i.p. The duration of hind paw licking/biting in the acetone test was recorded 3 days before (PRE) and at several days after the first injection of paclitaxel or its vehicle. Each animal was tested only in one nociceptive model. Each point and vertical line represents the mean ± S.E.M. of the values obtained in at least 12 mice. Statistically significant differences between the values of paclitaxel- and vehicle-treated groups: * *p* < 0.05; ** *p* < 0.01; and between the values on pre-treatment day and the days after treatment: # *p* < 0.05; ## *p* < 0.01 (two-way repeated measures ANOVA followed by Newman-Keuls test).

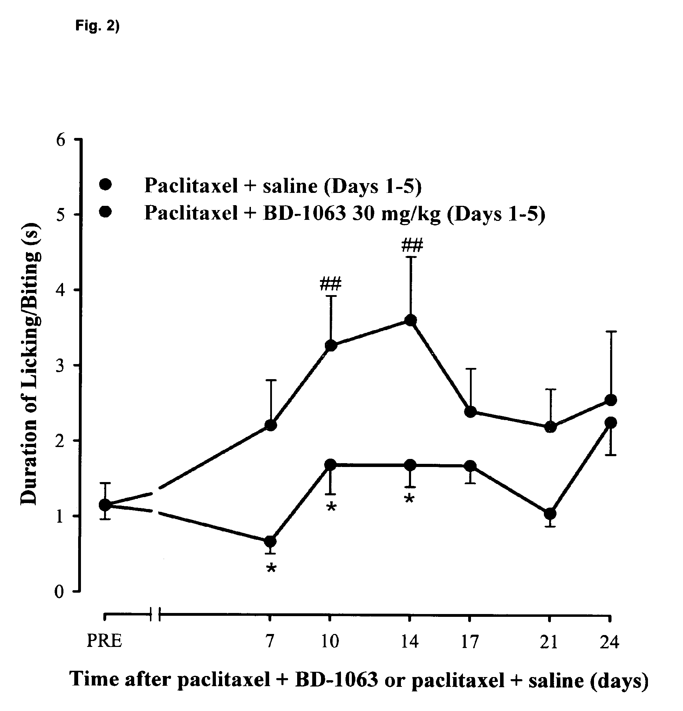

**Fig. 2**: Time-course of the effect of co-administration of paclitaxel + BD-1063 (30 mg/kg) or paclitaxel + saline on duration of hind paw licking/biting in the acetone test. Mice were treated once daily from days 1 to 5 with an i.p. co-injection of BD-1063 (30 mg/kg) and paclitaxel (2 mg/kg). The response evaluated was recorded in each animal 3 days before (PRE) and at several days after the first injection of paclitaxel + BD-1063 or paclitaxel + saline. Each animal was tested only in one nociceptive model. Each point and vertical line represents the mean ± S.E.M. of the values obtained in at least 16 animals. No statistically significant differences between the values of both groups of treatment were found in the plantar test, but only a slight tendency can be seen. Statistically significant differences in comparison to paclitaxel + saline: * *p* < 0.05, ** *p* < 0.01; and between the values on pre-treatment day and the days after treatment: # *p* < 0.05, ## *p* < 0.01 (two-way repeated measures ANOVA followed by Newman-Keuls test).

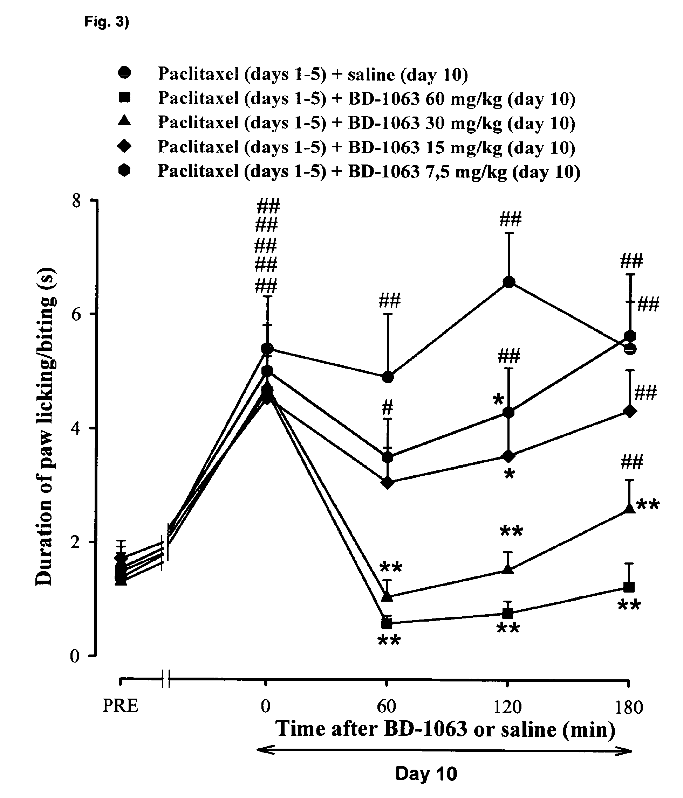

**Fig. 3**: Effect of a single treatment of various amounts of BD-1063 or saline on the duration of hind paw licking/biting (acetone test) on day 10 (a day of maximum effect) in mice pre-treated with paclitaxel. Animals were treated once daily from days 1 to 5 with paclitaxel or its vehicle via i.p. and the day 10 received a single injection of BD-1063 (7.5, 15, 30 and 60 mg/kg) or saline. The duration of hind paw licking/biting was recorded in each animal 3 days before (PRE) and 10 days after the first injection of paclitaxel or its vehicle. This day, duration of hind paw licking/biting was recorded immediately before (time 0) and at several times (60, 120 and 180 min) after the injection of TTX or saline. Each animal received either saline or one dose of BD-1063. Each point and vertical line represents the mean ± S.E.M. of the values obtained in at least 12 animals.

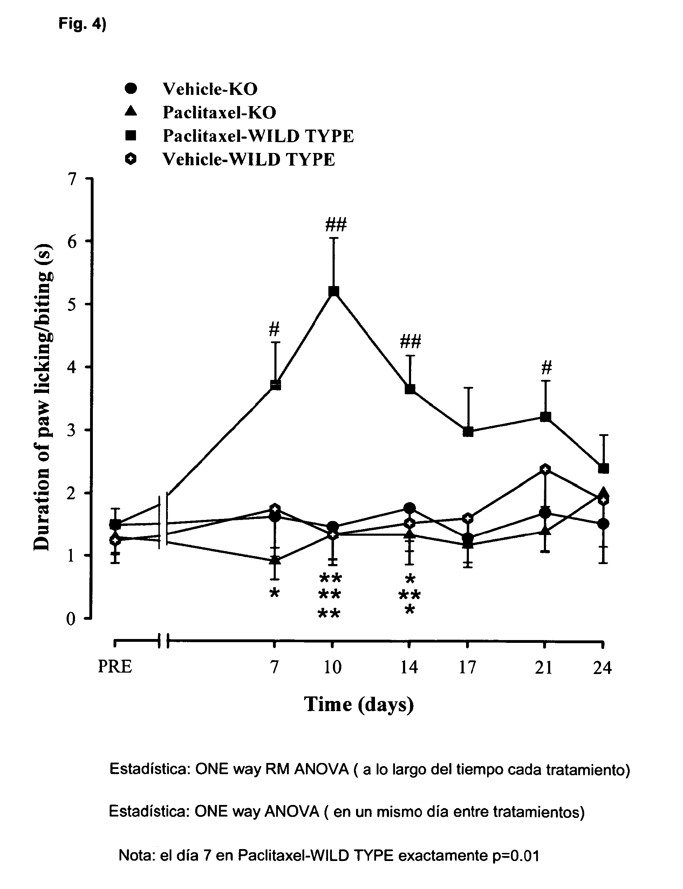

**Fig. 4**: Time-course of paclitaxel induced cold-allodynia in to groups of mice, one being sigma-1-receptor knock-out mice and the other wild-type mice. Animals were treated once daily from days 1 to 5 with paclitaxel (2 mg/kg) or its vehicle via i.p. The duration of hind paw licking/biting in the acetone test was recorded 3 days before (PRE) and at several days after the first injection of paclitaxel or its vehicle. Each animal was tested only in one nociceptive model. It turned out that only wild-type animals treated with paclitaxel showed increased duration of hind paw licking/biting in the acetone test. Each point and vertical line represents the mean ± S.E.M. of the values obtained in at least 12 mice. Statistically significant differences between the values of paclitaxel- and vehicle-treated groups: * *p* < 0.05; ** *p* < 0.01; and between the values on pre-treatment day and the days after treatment: # *p* < 0.05; ## *p* < 0.01 (two-way repeated measures ANOVA followed by Newman-Keuls test).

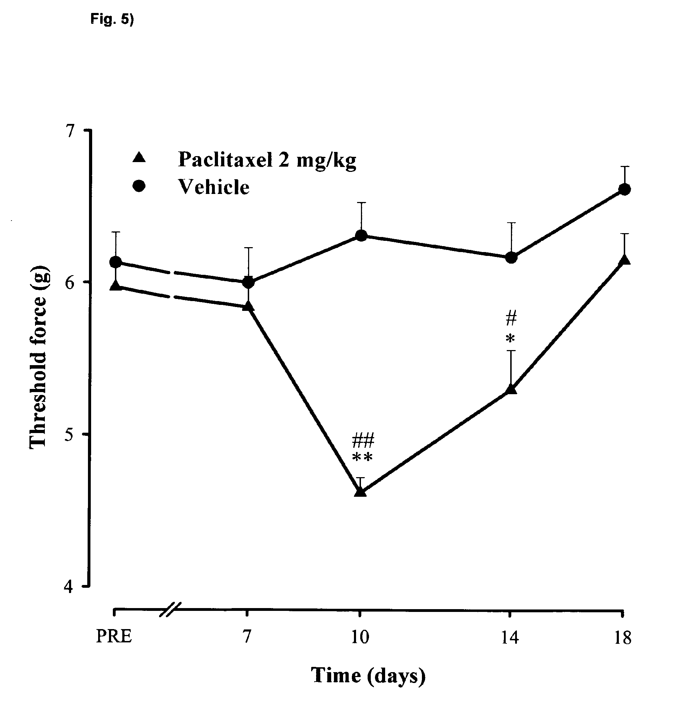

**Fig. 5**: Time-course of paclitaxel induced mechanical allodynia in mice. Animals were treated once daily from days 1 to 5 with paclitaxel (2 mg/kg) or its vehicle via i.p. The treshold force in the Von-Frey-test was recorded 3 days before (PRE) and at several days after the first injection of paclitaxel or its vehicle. Each animal was tested only in one nociceptive model. Each point and vertical line represents the mean ± S.E.M. of the values obtained in at least 12 mice. Statistically significant differences between the values of paclitaxel- and vehicle-treated groups: * *p* < 0.05; ** *p* < 0.01; and between the values on pre-treatment day and the days after treatment: # *p* < 0.05; ## *p* < 0.01 (two-way repeated measures ANOVA followed by Newman-Keuls test).

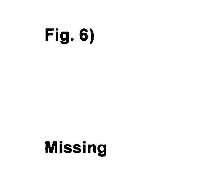

**Fig. 6**: Time-course of the effect of co-administration of paclitaxel + BD-1063 (30 mg/kg) or paclitaxel + saline on threshold force for hind paw withdrawal in the Von Frey test. Mice were treated once daily from days 1 to 5 with an i.p. co-injection of BD-1063 (30 mg/kg) and paclitaxel (2 mg/kg). The response evaluated was recorded in each animal 3 days before (PRE) and at several days after the first injection of paclitaxel + BD-1063 or paclitaxel + saline. Each animal was tested only in one nociceptive model. Each point and vertical line represents the mean ± S.E.M. of the values obtained in at least 16 animals. No statistically significant differences between the values of both groups of treatment were found in the plantar test, but only a slight tendency can be seen. Statistically significant differences in comparison to paclitaxel + saline: * *p* < 0.05, ** *p* < 0.01; and between the values on pre-treatment day and the days after treatment: # *p* < 0.05, ## *p* < 0.01 (two-way repeated measures ANOVA followed by Newman-Keuls test).

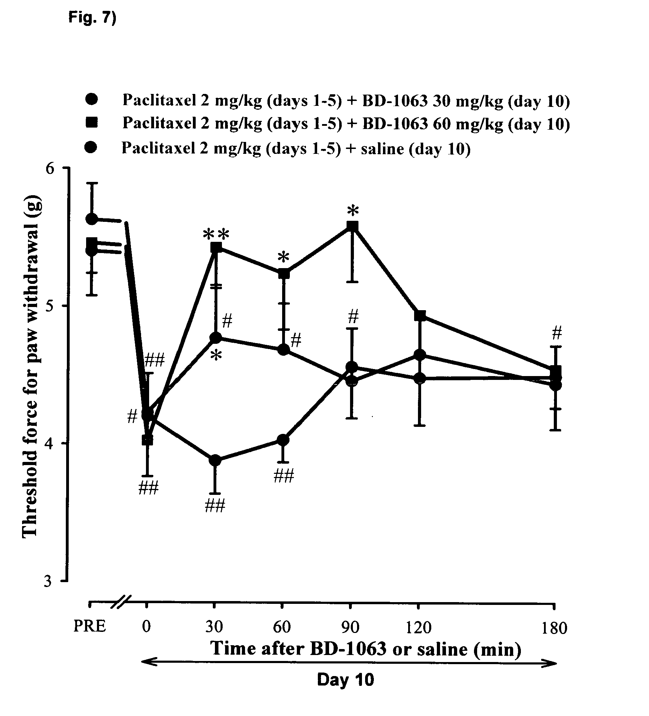

**Fig. 7**: Effect of a single treatment of various amounts of BD-1063 or saline on the threshold strength (von-Frey test) on day 10 (a day of maximum effect) in mice pre-treated with paclitaxel. Animals were treated once daily from days 1 to 5 with paclitaxel or its vehicle via i.p. and the day 10 received a single injection of BD-1063 (7.5, 15, 30 and 60 mg/kg) or saline. The threshold strength was recorded in each animal 3 days before (PRE) and 10 days after the first injection of paclitaxel or its vehicle. This day, threshold strength was recorded immediately before (time 0) and at several times (60, 120 and 180 min) after the injection of TTX or saline. Each animal received either saline or one dose of BD-1063. Each point and vertical line represents the mean ± S.E.M. of the values obtained in at least 12 animals.

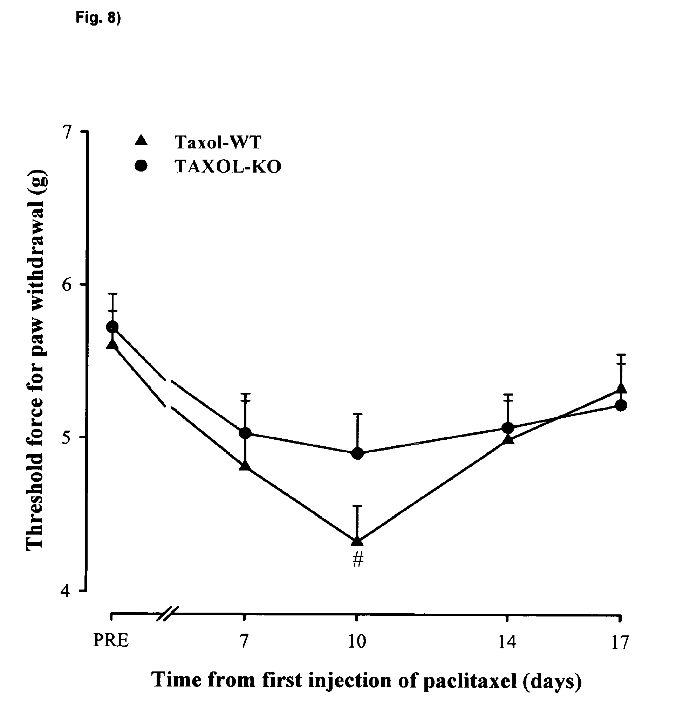

**Fig. 8**: Time-course of paclitaxel induced mechanical-allodynia in to groups of mice, one being sigma-1-receptor knock-out mice and the other wild-type mice. Animals were treated once daily from days 1 to 5 with paclitaxel (2 mg/kg) or its vehicle via i.p. The threshold force in the von-Frey-test was recorded 3 days before (PRE) and at several days after the first injection of paclitaxel or its vehicle. Each animal was tested only in one nociceptive model. It turned out that only wild-type animals treated with paclitaxel showed decreased threshold strength in the von-Frey test. Each point and vertical line represents the mean ± S.E.M. of the values obtained in at least 12 mice. Statistically significant differences between the values of paclitaxel- and vehicle-treated groups: * *p* < 0.05; ** *p* < 0.01; and between the values on pre-treatment day and the days after treatment: # *p* < 0.05; ## *p* < 0.01 (two-way repeated measures ANOVA followed by Newman-Keuls test).

### METHODS:

### In general:

Experiments were performed in CD-1 mice (Charles River, S.A.) with n = 10/experimental group. Paclitaxel-induced painful peripheral neuropathy was produced by i.p. administration of paclitaxel once daily during 5 days. Control animals received the same volume of solvent (a mixture of ethanol and cremophor EL)

Mechanical hyperalgesia and allodynia was evaluated with Von Frey filaments and a Randall-Sellito paw-pressure analgesymeter (Dina et al., 2001; Flatters and Bennet, 2004), and cold allodynia was evaluated using the acetone drop method (Polomano et al., 2001; Smith et al., 2004).

A well-known sigma receptor antagonist, BD-1063, was injected s.c. either immediately before each paclitaxel injection to test whether a Sigma-Antagonist effects the development of the painful peripheral neuropathy or in the third week (when paclitaxel injection have ended and the neuropathy is fully developed) to test whether BD-1063 interferes with the expression of the different signs of paclitaxel-induced neuropathic pain. In addition, to study the influence of the sigma 1 receptor in this process the difference in development of allodynia was determined using wild-type mice and sigma 1-receptor knock-out-mice.

### Specific description:

Mice weighing 25-30 g were used. The animals were housed in colony cages with free access to food and water prior to the experiments. They were maintained in temperature- and light-controlled rooms (22 ± 1 °C, lights on at 08.00 h and off at 20.00 h, air replacement every 20 min). Testing took place during the light phase (from 9.00 h to 15.00 h).

Paclitaxel was dissolved in a solution made up of 50% Cremophor EL and 50% absolute ethanol to obtain a concentration of 6 mg/ml. This paclitaxel solution was conserved at -20 °C during a maximum of 14 days and was diluted in normal saline (NaCl 0.9%), just before administration, to a final concentration of 2 mg/10 ml. The vehicle of paclitaxel was diluted at the time of injection with saline (NaCl 0.9%) in the same proportion as the paclitaxel solution.

Paclitaxel (2 mg/kg) was administered intraperitoneously (i.p.), in a volume of 10 ml/kg, once per day for five consecutive days. Therefore, the cumulative dose was 10 mg/kg per mouse. In the control group the vehicle of paclitaxel was administered following the same schedule. The same schedules of paclitaxel injection also applied when testing groups of Sigma-1-knock-out mice against groups of wild-type mice.

BD-1063 was dissolved in normal saline just before administration and applied at concentrations of 7.5 mg/kg, 15 mg/kg, 30 mg/kg and 60 mg/kg.

The effects of BD-1063 on paclitaxel-induced neuropathic pain were examined in two different ways. To evaluate the effect of the sigma receptor antagonist BD-1063 on the *development* of paclitaxel-induced pain, the animals received an injection of BD-1063 together with each i.p. injection of paclitaxel for five consecutive days. Posteriorly the response of the animals to the different nociceptive stimuli was tested during 2-4 weeks, depending on the test (see below), without any additional treatment. Each animal was tested only in one nociceptive model. To test the effect of BD-1063 on the *expression* of paclitaxel-induced pain a single BD-1063 injection was performed on day 10, a day of maximum expression of mechanical allodynia or cold allodynia (see figures for details). Each animal received only one dose of BD-1063 and was tested in only one nociceptive model.

**Procedure for assessment of cold allodynia.** Cold allodynia was tested as previously described by Smith et al., 2004, by gently touching the plantar skin of the hind paws with an acetone bubble formed with a syringe connected to a thin polyethylene tube. The mice were housed and habituated for 30 min in transparent plastic boxes (7 x 7 x 13 cm) with a floor made of wire mesh. After the adaptation period, acetone was applied alternately three times to each paw at intervals of 30 s, and the duration and frequency of licking or biting were recorded. A small mirror was placed behind the chambers to allow clear observation of the paws. The time spent licking or biting the paw was recorded by a stopwatch and represented as the cumulative time of licking/biting in the six measurements. Since in the experiments licking persisting more than 10 s was very unusual, a cut-off time of 10 s was used to each trial.

To elucidate the time-course of paclitaxel-induced cold allodynia in control mice, the animals were tested previously to paclitaxel administration (pretreatment value, 3 days before first paclitaxel-treatment) and on different days (days 7, 10, 14, 17, 21 and 24) after the first paclitaxel or vehicle injection.

The same procedure was followed to compare Sigma-1-knock-out mice to wilde type mice, thus elucidating the time-course of paclitaxel-induced cold allodynia in control mice. Accordingly, the animals (two equal groups of knock-out and wild-type mice were tested previously to paclitaxel administration (pretreatment value, 3 days before first paclitaxel-treatment) and on different days (days 7, 10, 14, 17, 21 and 24) after the first paclitaxel or vehicle injection.

The same procedure was followed to test the effect of BD-1063 on the development of cold allodynia, but in this case, BD-1063 or its vehicle was injected together with each of the 5 paclitaxel i.p. injections. Again the animals were tested previously to paclitaxel/BD-1063 administration (pretreatment value, 3 days before first paclitaxel/BD-1063-treatment) and on different days (days 7, 10, 14, 17, 21 and 24) after the first paclitaxel/BD-1063 or vehicle injection. The effect of BD-1063 on the expression of paclitaxel-induced cold allodynia was evaluated on day 10, because the maximum allodynic effect was observed on that day. Therefore, the day 10, after the habituation period to the apparatus, baseline latencies were recorded, 30 min later BD-1063 or saline was injected s.c. and paw withdrawal latencies were assessed again 30, 60, 90, 120 and 180 minutes after the injection. Around 33 % of the control animals treated with paclitaxel did not show cold allodynia; therefore, it was differentiated between 'responders' and 'non-responders' mice in this test. The 'non-responders' mice were easily identified because they spent less than 2 s licking/biting the paw stimulated with acetone on days 7 and 10 after paclitaxel administration. The 'non-responder' animals were not used to test the effect of BD-1063 on the expression of cold allodynia since they do not express enough cold allodynia.

**Procedure for assessment of mechanical allodynia**. To assess mechanical allodynia, paw withdrawal thresholds were measured using a Dynamic Plantar Aesthesiometer (Ugo Basile, Italy). The electronic Von Frey device employs a single nonflexible filament which applies a progressively increasing force (from 0 to 10 g) against the plantar surface of the hind paw over a 20 s period. The nocifensive withdrawal reflex automatically turns off the stimulus and the mechanical threshold value is showed in a screen. The day of the experiment, mice were placed individually in test compartments (9 x 9 x 14 cm) with a wire mesh bottom and allowed to acclimatize to them for 2 h. After habituation, each mouse was tested three times alternately in each hind paw.

To elucidate the time-course of paclitaxel-induced mechanical allodynia in control mice, the animals were tested previously to paclitaxel administration (pretreatment value; day 3 before paclitaxel-treatment) and on different days (days 7, 10, 14 and 17) after the first paclitaxel or vehicle injection.

The same procedure was followed to compare Sigma-1-knock-out mice to wilde type mice, thus elucidating the time-course of paclitaxel-induced mechanical allodynia in control mice. Accordingly, the animals (two equal groups of knock-out and wild-type mice were tested previously to paclitaxel administration (pretreatment value, 3 days before first paclitaxel-treatment) and on different days (days 7, 10, 14, 17, 21 and 24) after the first paclitaxel or vehicle injection.

The same procedure was followed to test the effect of BD-1063, a well-know sigma-receptor antagonist, on the development of paclitaxel-induced mechanical allodynia. in this case, BD-1063 or its vehicle was injected together with each of the 5 paclitaxel i.p. injections. Again the animals were tested previously to paclitaxel/BD-1063 administration (pretreatment value, 3 days before first paclitaxel/BD-1063-treatment) and on different days (days 7, 10, 14, 17, 21 and 24) after the first paclitaxel/BD-1063 or vehicle injection. The effect of BD-1063 on the expression of paclitaxel-induced mechanical allodynia was evaluated on day 10, because the maximum change of the mechanical threshold was observed on that day. Therefore, the day 10, after the habituation period to the apparatus, baseline latencies were recorded, 30 min later BD-1063 or saline was injected s.c. and paw withdrawal latencies were assessed again 30, 60, 90, 120 and 180 minutes after the injection. Most animals (96 %) treated with paclitaxel showed a reduction of the mechanical threshold; those animals that did not show mechanical allodynia were not used to test the effect of BD-1063 on the expression of paclitaxel-induced mechanical allodynia.

### Results

**A) Time-course of paclitaxel-induced cold- and mechanical-allodynia in control mice**. The values obtained on the pre-treatment day in paclitaxel- and vehicle-treated animals were not significantly different in acetone test and Von Frey test. Administration during 5 days of paclitaxel-vehicle did not significantly modify the response of the animals in any test at any post-treatment day in comparison to the pre-treatment value.

In the acetone test (Fig. 1), administration of paclitaxel (2 mg/kg, i.p.) once daily during 5 days permits to distinguish two groups of animals depending on their response. Most animals (67%) treated with paclitaxel significantly (p < 0.01) increased the time spent licking/biting the paw stimulated (Fig. 1) and the frequency of paw licking/biting all the post-treatment days, in comparison to the pre-treatment day value. These animals constitute the paclitaxel-responder animals. On the other hand, a 33% of paclitaxel-treated animals did not show cold allodynia, and their response to acetone was indistinguishable from that of animals treated with paclitaxel-vehicle in both duration (Fig. 1) and frequency of licking/biting. When the values of these two variables among the different groups on the same day of evaluation were compared, statistically significant differences between paclitaxel-responder and the other two groups (paclitaxel-non-responder or paclitaxel-vehicle) each day of evaluation after treatment (Fig. 1) was observed. Paclitaxel-induced cold allodynia was maximal 10-14 days after the first injection of the antineoplastic for both variables recorded (Fig. 1); therefore, the effect of BD-1063 on the expression of cold allodynia was evaluated on day 10.

Administration of paclitaxel (2 mg/kg, i.p., during 5 days) induced mechanical allodynia in mice, since it significantly reduced the threshold force for paw withdrawal in the Von Frey test on day 10, in comparison both with the pre-treatment day value and with the value obtained the same day in the paclitaxel-vehicle treated animals (Fig. 5). Therefore, the effect of TTX on the expression of mechanical allodynia was tested on day 10.

**B) Effect of BD-1063 on the development of paclitaxel-induced cold- and mechanical-allodynia.** The pre-treatment values were similar in the two experimental groups (paclitaxel + saline and paclitaxel + BD-1063) in all tests performed (acetone and Von Frey tests).

The group of animals in which paclitaxel (i.p.) and saline (s.c.) were co-administered during days 1 to 5 showed a significant increase on duration of paw licking/biting (Fig. 2) in the acetone test, which began on day 7 and became maximum on days 10-14 after the first injection, like in the experiments were only paclitaxel was injected (Fig. 1). On the other hand, in the animals which receive an i.p. co-injection of BD-1063 in a dose of 30 mg/kg together with paclitaxel during days 1 to 5, the duration of paw licking/biting (throughout the 24 days after co-administration) were statistically significantly different between the values obtained in both groups (paclitaxel + saline and paclitaxel + BD-1063 (30mg/kg)) from day 7 onwards when duration of licking/biting was analysed. Therefore, co-administration of paclitaxel i.p. and BD-1063 (30 mg/kg) inhibited the development of cold allodynia induced by paclitaxel.

The paw withdrawal threshold force values (Von Frey test) in paclitaxel + saline treated animals were significantly lower than the pre-treatment values on days 10 and 14 after the first injection of paclitaxel (Fig. 6). There were statistically significant differences between the values of paw withdrawal threshold force after the co-administration of paclitaxel with BD-1063 (30 mg/kg) in comparison to that obtained after paclitaxel with saline on days 10 and 14 (Fig. 6). Therefore, co-administration of paclitaxel and BD-1063 inhibited the development of mechanical allodynia induced by paclitaxel in mice.

**C) Effect of BD-1063 on the expression of paclitaxel-induced cold allodynia.** The duration and the frequency of paw licking/biting on day 10, before the treatment with BD-1063 or saline, were significantly different from their values on pre-treatment day in all groups of animals treated.As expected, paclitaxel induced a cold allodynia 10 days after its first injection. A single s.c. injection of saline on day 10 did not significantly modify the expression of paclitaxel-induced cold allodynia. The acute treatment with various amounts of BD-1063 (7.5, 15, 30 and 60 mg/kg) inhibited the expression of paclitaxel-induced cold allodynia. This effect of BD-1063 was dose-dependently, significantly different from that of saline (Fig.3).

**D) Effect of BD-1063 on the expression of paclitaxel-induced mechanical allodynia.** The threshold strength on day 10, before the treatment with BD-1063 or saline, were significantly different from their values on pre-treatment day in all groups of animals treated. As expected, paclitaxel induced mechanical allodynia 10 days after its first injection. A single s.c. injection of saline on day 10 did not significantly modify the expression of paclitaxel-induced mechanical allodynia. The acute treatment with various amounts of BD-1063 (7.5, 15, 30 and 60 mg/kg) inhibited the expression of paclitaxel-induced mechanical allodynia. This effect of BD-1063 was dose-dependently, significantly different from that of saline (Fig.7).

**E) Time-course of paclitaxel-induced cold-allodynia in a comparison between wild-type mice and sigma-1-receptor wild-type mice**. In the acetone test (Fig. 4), administration of paclitaxel (2 mg/kg, i.p.) once daily during 5 days resulted in two different effects in the two groups of animals. In wild-type animals paclitaxel-induced cold allodynia was maximal 10-14 days after the first injection of the antineoplastic for both variables recorded (Fig. 4), whereas no effect was to be seen with saline. In Sigma-1-receptor animals paclitaxel-induced cold allodynia was not significantly expressed, neither in the sub-group treated with paclitaxel, nor in the saline treated group (Fig. 4). Therefore, the effect paclitaxel induced cold allodynia is an effect related to sigma-1.

**F) Time-course of paclitaxel-induced mechanical-allodynia in a comparison between wild-type mice and sigma-1-receptor wild-type mice.** In the von-Frey test (Fig. 8), administration of paclitaxel (2 mg/kg, i.p.) once daily during 5 days resulted in two different effects in the two groups of animals. In wild-type animals paclitaxel-induced mechanical allodynia was maximal 10-14 days after the first injection of the antineoplastic for both variables recorded (Fig. 8), whereas no effect was to be seen with saline. In Sigma-1-receptor animals paclitaxel-induced mechanical allodynia was not significantly expressed, neither in the sub-group treated with paclitaxel, nor in the saline treated group (Fig. 4). Therefore, the effect paclitaxel induced mechanical allodynia is an effect related to sigma-1.

## Claims

1. Use of a compound binding to the sigma receptor for the treatment or prevention of pain developing as a consequence of chemotherapy.

2. Use according to claim 1 in which the compound binding to the sigma receptor is used for the prevention of the development of pain developing as a consequence of chemotherapy.

3. Use according to claim 1 in which the compound binding to the sigma receptor is used for the treatment of pain developing as a consequence of chemotherapy.

4. Use, according to Claim 3, in which the pain is either neuropathic pain, allodynia or hyperalgesia.

5. Use, according to Claim 4, in which the neuropathic pain is selected from peripheral neuropathic pain, allodynia, causalgia, hyperalgesia, hyperesthesia, hyperpathia, neuralgia, neuritis or neuropathy.

6. Use, according to Claim 1, **characterized in that** the compound may be in neutral form, in the form of a base or acid, in the form of a salt, preferably a physiologically acceptable salt, in the form of a solvate or of a polymorph and/or in the form of in the form of its racemate, pure stereoisomers, especially enantiomers or diastereomers or in the form of mixtures of stereoisomers, especially enantiomers or diastereomers, in any suitable mixing ratio.

7. Use, according to claim 1, **characterized in that** said compound binding to the sigma receptor has an IC50 value of ≤ 5000 nM, preferably **in that** said compound binding to the sigma receptor has an IC50 value of ≤1000 nM, more preferably **in that** said compound binding to the sigma receptor has an IC50 value of ≤500 nM, most preferably **in that** said compound binding to the sigma receptor has an IC50 value of ≤250 nM.

8. Use, according to claim 1, **characterized in that** said compound binding to the sigma receptor has an IC50 value of ≤100 nM, most preferably **in that** said compound binding to the sigma receptor has an IC50 value of ≤50 nM.

9. Use, according to any of claims 1 to 6, **characterized in that** said compound binding to the sigma receptor used is binding to the sigma receptor as an antagonist, a partial antagonist or an inverse agonist.

10. Use, according to any of claims 1 to 9, **characterized in that** said compound binding to the sigma receptor is binding to the sigma-1 receptor subtype.

11. Use, according to claim 1, **characterized in that** said compound binding to the sigma receptor is selected from the group consisting of:
| | |
|---|---|
| (-)-Cyanopindolol hemifumarate | (-)-SPARTEINE SULFATE PENTAHYDRATE |
| (+)-HIMBACINE | (2-Dibutylamino-Ethyl)-Carbamic Acid 2-(4-Benzofuran-2-Ylmethyl-Piperazin-1-Yl)-Ethyl Ester |
| (4-[1,2,3]Thiadiazol-4-Yl-Benzyl)-Carbamic Acid 1-(3-Methoxy-2-Nitro-Benzyl)-Piperidin-3-Ylmethyl Ester | (S)-Methamphetamine HCl |
| [1-(9-Ethyl-9H-Carbazol-3-Ylmethyl)-Pyrrolidin-3-Yl]-Carbamic Acid 1-(3-Benzyloxy-4-Methoxy-Benzyl)-Piperidin-3-Ylmethyl Ester | [1-(9-Ethyl-9H-Carbazol-3-Ylmethyl)-Pyrrolidin-3-Yl]-Carbamic Acid 2-(Tert-Butoxycarbonyl-Naphthalen-1-Ylmethyl-Amino)-Ethyl Ester |
| [4-(4-Ethyl-3,5-Dimethyl-Pyrazol-1-Yl)-Phenyl]-[4-(3-Phenyl-Allyl)-Piperazin-1-YI]-Methanone | 1-(1,2-Diphenylethyl)Piperidine Maleate, (+/-) |
| 1-(1-Naphthyl)Piperazine HCl | 1-(3-Chlorophenyl)Piperazine HCl |
| 1-(4-Bromo-Benzenesulfonyl)-4-(2-Tert-Butylsulfanyl-Benzyl)-Piperazine | 2-(2-{[1-(3-Chloro-Benzyl)-Pyrrolidin-3-Yl]-Methyl-Carbamoyl)-2-Methyl-Propyl)-4,6-Dimethyl-Benzoic Acid |
| 2-Chloro-11-(4-Methylpiperazino)Dibenz[B,F]Oxepin Maleate | 3,3'-Diethylthiacarbocyanine Iodide |
| 3-Mercapto-2-Methylpropanoic Acid 1,2-Diphenylethylamine Salt | 3-Quinuclidinyl Benzilate |
| 3-Tropanyl-3,5-Dichlorobenzoate | 3-Tropanyl-Indole-3-Carboxylate HCl |
| 4-(1H-indol-4-Yl)-Piperazine-1-Carboxylic Acid 2-(5-Bromo-2-Ethoxy-Phenylamino)-Cyclohexylmethyl Ester | 4-(2-Tert-Butylsulfanyl-Benzyl)-Piperazine-1-Carboxylic Acid 2-Thiophen-2-Yl-Ethyl Ester |
| 4-(3,5-Dimethoxy-Phenyl)-Piperazine-1-Carboxylic Acid 1-(2-Fluoro-Benzyl)-Piperidin-2-Ylmethyl Ester | 4-(3-Nitro-5-Sulfamoyl-Thiophen-2-Yl)-Piperazine-1-Carboxylic Acid 1-(2-Fluoro-5-Methoxy-Benzyl)-Piperidin-3-Ylmethyl Ester |
| 4-(4-Fluorobenzoyl)-1-(4-Phenylbutyl)Piperidine Oxalate | 4-(5-Trifluoromethyl-Pyridin-2-Yl)-Piperazine-1-Carboxylic Acid Pent-2-Ynyl Ester |
| 4,4'-Bis[4-(P-Chlorophenyl)-4-Hydroxypiperidino]Butyrophenone | 4-[1-(4-Chlorobenzyl)-4-(benzylpiperidin-4-yl]-2-hydroxy-4-oxobut-2-enoic acid |
| 4-Bromo-N-[1-(9-Ethyl-9H-Carbazol-3- | 4'-Chloro-3-Alpha- |
| Ylmethyl)-Pyrrolidin-3-Yl]-2-Trifluoromethoxy-Benzenesulfonamide | (Diphenylmethoxy)Tropane HCl |
| 4-Furan-2-Ylmethyl-Piperazine-1-Carboxylic Acid 2-{4-[3-(2-Trifluoromethyl-Phenothiazin-10-Yl)-Propyl]-Piperazin-1-Yl}-Ethyl Ester | 4-Methoxy-N-[1-(7-Methoxy-Benzo[1,3]Dioxol-5-Ylmethyl)-Pyrrolidin-3-Yl]-Benzenesulfonamide |
| 5-(N-Ethyl-N-Isopropyl)-Amiloride | 7-Hydroxy-DPAT HBr, (±)- |
| 8-Hydroxy-DPAT HBr, (R)-(+)- | 8-Hydroxy-DPAT HBr, S(-)- |
| 9-[4-({[4'-(trifluoromethyl)-1,1'-biphenyl-2-yl]carbonyl}amino)piperidin-1-yl]-N-(2,2,2-trifluoroethyl)-9H-fluorene-9-carboxamide | Acepromazine Maleate |
| Acetophenazine Maleate | Acrinol |
| Ajmaline | Alaproclate HCl |
| Aloe-Emodin | Alprenolol D-Tartrate Salt Hydrate |
| Alprenolol HCl | AMI-193 |
| Aminobenztropine | Amiodarone HCl |
| Amodiaquine HCl | Amorolfine HCl |
| Amoxapine | Anileridine HCl |
| Anisotropine Methylbromide | Anpirtoline |
| ARC 239 DiHCl | Astemizole |
| Auramine O HCl | Azaperone |
| Azatadine Maleate | Azelastine HCl |
| Bamethan sulfate | BD 1008 DiHBr |
| BD-1047 | BD-1063 |
| Benextramine TetraHCl | Benfluorex HCl |
| Benidipine HCl | Benoxathian HCl |
| Benoxinate HCl | Benperidol |
| Benproperine Phosphate | Benzododecinium bromide |
| Benzphetamine HCl | Benztropine Mesylate |
| Benzydamine HCl | Bephenium Hydroxynaphthoate |
| Bepridil HCl | Berberine chloride |
| Betaxolol HCl | Bifemelane |
| BMY 7378 DiHCl | Bopindolol Malonate |
| BP 554 Maleate | Bromhexine HCl |
| Bromodiphenhydramine HCl | Bromperidol |
| Brompheniramine Maleate | BTCP HCl |
| Buclizine HCl | Buflomedil HCl |
| Bupropion HCl | Buspirone HCl |
| Butacaine Sulfate | Butaclamol HCl, (±)- |
| Butenafine HCl | Butoconazole Nitrate |
| BW 723C86 HCl | Carbetapentane Citrate |
| Carbinoxamine Maleate | Carpipramine DiHCl DiH2O |
| Carvedilol | Cephapirin Benzathine |
| CGS-12066A Maleate | Chloroprocaine HCl |
| Chloroquine Phosphate | Chlorpheniramine Maleate |
| Chlorphenoxamine HCl | Chlorpromazine HCl |
| Chlorprothixene | Cinanserin HCl |
| Cinnarizine | Cirazoline HCl |
| Cis-(+/-)-N-Methyl-N-[2-(3,4-Dichlorophenyl)Ethyl]-2-(1-Pyrrolidinyl)Cyclohexamine DiHBr | Cis(Z)-Flupentixol DiHCl |
| Cisapride Hydrate | Citalopram HBr |
| Clebopride Maleate Salt | Clemastine Fumarate |
| Clemizole HCl | Clenbuterol HCl |
| Clidinium Bromide | Clobenpropit 2HBr |
| Clofazimine | Clofilium Tosylate |
| Clomiphene Citrate | Clomiphene Related Compound A |
| Clomipramine | Cloperastine HCl |
| Clorgyline HCl | Clozapine |
| CONESSINE | Cyclizine |
| Cyclobenzaprine HCl | Cycloheximide |
| Cyproheptadine HCl | Darrow Red HC |
| Demecarium Bromide | Denatonium Benzoate |
| Deptropine Citrate | Desloratadine |
| Dexbrompheniramine Maleate | Dexchlorpheniramine Maleate |
| Dexfenfluramine HCl | Dibucaine HCl |
| Dicyclomine HCl | Diethylpropion HCl |
| Dimethisoquin HCl | Dimetindene Maleate |
| Diphemanil Methylsulfate | Diphenidol HCl |
| Diphenoxylate HCl | Diphenylpyraline HCl |
| Dipropyldopamine HBr | Dobutamine HCl |
| Donepezil HCl | Doxepin HCl |
| Droperidol | Duloxetine |
| Dyclonine HCl | Ebastine |
| Econazole Nitrate | Epinastine HCl |
| Ethaverine HCl | Ethopropazine HCl |
| Eticlopride HCl, S(-)- | Etofenamate |
| Etonitazenyl Isothiocyanate | Femoxetine HCl |
| Fenfluramine HCl | Fentanyl Citrate |
| Fenticonazole Nitrate | Fipexide HCl |
| Flavoxate HCl | Flunarizine diHCl |
| Fluoxetine Related Compound B | Fluperlapine |
| Fluphenazine Decanoate DiHCl | Fluphenazine Enanthate DiHCl |
| Fluphenazine HCl | Fluphenazine N-Mustard DiHCl |
| Flurazepam Related Compound C | Fluspirilene |
| Fluvoxamine Maleate | GBR 12783 DiHCl |
| GBR 12909 DiHCl | GBR 13069 DiHCl |
| GBR-12935 DiHCl | GR 89696 Fumarate |
| Guanabenz Acetate | Guanadrel Sulfate |
| Guanethidine Sulfate | Halofantrine HCl |
| Haloperidol | HEAT HCl |
| Hexylcaine HCl | Hycanthone |
| Hydroxychloroquine Sulfate | Hydroxyzine HCl |
| Hyoscyamine Sulfate | IBZM, S(-)- |
| ICI-199,441 HCl | Ifenprodil Tartrate |
| Imipramine HCl | Indatraline HCl |
| lofetamine HCl | Irinotecan HCl |
| Isamoltane Hemifumarate | Isopromethazine HCl |
| Isoxsuprine HCl | Ketanserin L-Tartrate |
| Ketoconazole | Ketotifen Fumarate Salt |
| L-693,403 Maleate | L-741,626 |
| L-741,742 HCl | L-745,870 TriHCl |
| Labetalol HCl | Levetimide HCl, R(-) |
| Levobunolol HCl | Lidoflazine |
| Lisuride Hydrogen Maleate, R(+)- | Lobeline HCl |
| lomerizine diHCl | Loperamide HCl |
| Loxapine Succinate | LY-53,857 Maleate |
| Maprotiline HCl | Mazindol |
| MDL 12,330A HCl | Mebhydroline 1,5-naphthalendisulfonate Salt |
| Meclizine HCl | Mefloquine HCl |
| Meprylcaine HCl | Mesoridazine Besylate |
| Metaphit Methanesulfonate | Metergoline |
| Methantheline Bromide | Methdilazine |
| Methiothepin Mesylate | Methixene HCl |
| Methoctramine | Methotrimeprazine Maleate |
| Methylene Violet 3Rax HCl | Metipranolol |
| Mexiletine HCl | Mianserin HCl |
| Miconazole | ML-9 HCl |
| Morantel Hydrogen L-Tartrate | MR 16728 HCl |
| N-(2-Chloroethyl)-N-Ethyl-2-Bromobenzylamine HCl | N'-[2-(Benzo[1,2,5]Thiadiazole-4-Sulfonylamino)-Acetyl]-Hydrazinecarboxylic Acid 2-(2-{4-[(4-Chloro-Phenyl)-Phenyl-Methyl]-Piperazin-1-Yl)-Ethoxy)-Ethyl Ester |
| Nafronyl Oxalate Salt | Naftifine |
| Naftopidil diHCl | Naltriben Mesylate |
| NAN-190 HBr | NE-100 |
| Nefazodone | Nefopam HCl |
| Nicardipine HCl | Nicergoline |
| Niguldipine HCl, (+/-)- | Nisoxetine HCl |
| Nortriptyline HCl | Nylidrin HCl |
| Octoclothepin Maleate, (±)- | Orphenadrine Citrate |
| Oxamniquine | Oxamniquine Related Compound A |
| Oxamniquine Related Compound B | Oxatomide |
| Oxiconazole Nitrate | Oxybutynin HCl |
| Panaxatriol | PAPP |
| Paroxetine | Paxilline |
| p-Chlorobenzhydrylpiperazine | Penbutolol Sulfate |
| Pentamidine Isethionate | Pentazocine, (±)- |
| Pergolide Methanesulfonate | Perhexiline Maleate Salt |
| Perospirone | Perphenazine |
| Perphenazine Sulfoxide | Phenamil Methanesulfonate |
| Phencyclidine HCl | Phenosafranin HCl |
| Phenoxybenzamine HCl | Phenyltoloxamine Citrate Salt |
| Piboserod | Pimozide |
| Pinacyanol Chloride | Pindobind, (+/-)- |
| Piperacetazine | Piperazine-1,4-Dicarboxylic Acid Benzyl Ester 2-[4-(4-Dimethylamino-Benzyl)-Piperazin-1-Yl]-Ethyl Ester |
| Piperidolate HCl | Pirenperone |
| PPHT HCl, (±)- | Pramoxine HCl |
| Prenylamine Lactate Salt | Pridinol Methanesulfonate Salt |
| Prochlorperazine Maleate | Procyclidine HCl |
| Proflavine Hemisulfate Salt | Progesterone |
| Promazine HCl | Promethazine HCl |
| Propafenone HCl | Proparacaine HCl |
| Propericyazine | Propiomazine |
| Propranolol HCl | Protokylol |
| Protriptyline HCl | Pyrilamine Maleate |
| Pyrimethamine | Pyrrolidine-1,2-Dicarboxylic Acid 1-[1-(4-Allyloxy-Benzyl)-Piperidin-2-Ylmethyl] Ester 2-Benzyl Ester |
| Pyrvinium Pamoate | Quetiapine Fumarate |
| Quinacrine HCl | Quinaldine Red |
| Quipazine Dimaleate | Quipazine, 6-Nitro-, Maleate |
| Raloxifene | Rimantadine HCl |
| Risperidone | Ritanserin |
| Ritodrine HCl | RS 23597-190 HCl |
| RS 67333 HCl | RS 67506 HCl |
| Safranin O HCl | Salmeterol |
| SB203186 | SCH-23390 HCl, R(+)- |
| Sertaconazole Nitrate | Sertindole |
| Sertraline | Sibutramine HCl |
| SKF-525A HCl | SKF-96365 HCl |
| SNC 121 | Spiperone HCl |
| Sufentanil | T-226296 |
| Tamoxifen Citrate | Tamsulosin HCl |
| Tegaserod Maleate | Terbinafine HCl |
| Terconazole | Terfenadine |
| Terfenadine Related Compound A | Tetracaine HCl |
| Tetrindole Mesylate | Thiethylperazine Malate |
| Thioperamide Maleate | Thioproperazine |
| Thioridazine | Thiothixene |
| Thiothixene, (E)- | Thonzonium Bromide |
| Tioconazole Related Compound A | TMB-8 HCl |
| Tolterodine L-Tartrate | Toremifene Citrate |
| Tramazoline HCl | Trans-U-50488 Methanesulfonate, (±)- |
| Trazodone HCl | Tridihexethyl Chloride |
| Trifluoperazine HCl | Trifluperidol HCl |
| Triflupromazine HCl | Trihexyphenidyl HCl |
| Trimebutine | Trimeprazine Hemi-L-Tartrate |
| Trimipramine Maleate | Tripelennamine HCl |
| Triprolidine HCl | Triprolidine HCl Z Isomer |
| Tropanyl 3,5-Dimethylbenzoate | Tropine 2-(4-Chlorophenoxy)Butanoate, Maleate |
| U-50488 HCl, (-)- | U-62066 |
| UH 232 Maleate, (+)- | Vecuronium Bromide |
| Verapamil HCl | Verapamil Related Compound B |
| Vesamicol HCl | Vinpocetine |
| W-7 HCl | WB-4101 HCl |
| Xylazine | Xylometazoline HCl |

12. Use, according to claim 11, **characterized in that** said compound binding to the sigma receptor is selected from the group consisting of:
| | |
|---|---|
| (2-Dibutylamino-Ethyl)-Carbamic Acid 2-(4-Benzofuran-2-Ylmethyl-Piperazin-1-Yl)-Ethyl Ester | (4-[1,2,3]Thiadiazol-4-YI-Benzyl)-Carbamic Acid 1-(3-Methoxy-2-Nitro-Benzyl)-Piperidin-3-Ylmethyl Ester |
| 4-(4-Fluorobenzoyl)-1-(4-Phenylbutyl)Piperidine Oxalate | 4-[1-(4-Chlorobenzyl)-4-(benzylpiperidin-4-yl]-2-hydroxy-4-oxobut-2-enoic acid |
| 4-Bromo-N-[1-(9-Ethyl-9H-Carbazol-3-Ylmethyl)-Pyrrolidin-3-Yl]-2-Trifluoromethoxy-Benzenesulfonamide | 4'-Chloro-3-Alpha-(Diphenylmethoxy)Tropane HCl |
| 4-Furan-2-Ylmethyl-Piperazine-1-Carboxylic Acid 2-{4-[3-(2-Trifluoromethyl-Phenothiazin-10-Yl)-Propyl]-Piperazin-1-Yl}-Ethyl Ester | Acetophenazine Maleate |
| Aminobenztropine | Amiodarone HCl |
| Amodiaquine HCl | Amorolfine HCl |
| Anileridine HCl | Astemizole |
| Azaperone | Azelastine HCl |
| BD 1008 DiHBr | BD-1047 |
| BD-1063 | Benextramine TetraHCl |
| Benfluorex HCl | Benoxathian HCl |
| Benperidol | Benproperine Phosphate |
| Benzododecinium bromide | Benztropine Mesylate |
| Bepridil HCl | Berberine chloride |
| Bifemelane | BP 554 Maleate |
| Bromhexine HCl | Bromodiphenhydramine HCl |
| Bromperidol | Buflomedil HCl |
| Butacaine Sulfate | Butaclamol HCl, (±)- |
| Butenafine HCl | Carbetapentane Citrate |
| Carpipramine DiHCl DiH2O | Cinnarizine |
| Cis-(+/-)-N-Methyl-N-[2-(3,4-Dichlorophenyl)Ethyl]-2-(1-Pyrrolidinyl)Cyclohexamine DiHBr | Cis(Z)-Flupentixol DiHCl |
| Cisapride Hydrate | Clofilium Tosylate |
| Clomiphene Citrate | Clomiphene Related Compound A |
| Clomipramine | Cloperastine HCl |
| Clorgyline HCl | Cyclobenzaprine HCl |
| Cyproheptadine HCl | Demecarium Bromide |
| Deptropine Citrate | Dibucaine HCl |
| Dicyclomine HCl | Diphenylpyraline HCl |
| Donepezil HCl | Doxepin HCl |
| Dyclonine HCl | Femoxetine HCl |
| Flunarizine diHCl | Fluphenazine Decanoate DiHCl |
| Fluphenazine Enanthate DiHCl | Fluphenazine HCl |
| Fluphenazine N-Mustard DiHCl | GBR 12783 DiHCl |
| GBR 12909 DiHCl | GBR 13069 DiHCl |
| GBR-12935 DiHCl | Haloperidol |
| HEAT HCl | Hexylcaine HCl |
| Hydroxyzine HCl | Ifenprodil Tartrate |
| lsopromethazine HCl | Isoxsuprine HCl |
| L-693,403 Maleate | L-741,626 |
| L-741,742 HCl | L-745,870 TriHCl |
| Lidoflazine | Lobeline HCl |
| lomerizine diHCl | Loperamide HCl |
| LY-53,857 Maleate | Metergoline |
| Methdilazine | Methixene HCl |
| Metipranolol | ML-9 HCl |
| MR 16728 HCl | Naftifine |
| Naftopidil diHCl | NAN-190 HBr |
| Nicardipine HCl | Nylidrin HCl |
| Octoclothepin Maleate, (±)- | Oxamniquine Related Compound A |
| Oxybutynin HCl | PAPP |
| Penbutolol Sulfate | Pentazocine, (±)- |
| Perphenazine | Phenoxybenzamine HCl |
| Pimozide | Piperidolate HCl |
| PPHT HCl, (±)- | Prenylamine Lactate Salt |
| Prochlorperazine Maleate | Promazine HCl |
| Proparacaine HCl | Protriptyline HCl |
| Pyrrolidine-1,2-Dicarboxylic Acid 1-[1-(4-Allyloxy-Benzyl)-Piperidin-2-Ylmethyl] Ester 2-Benzyl Ester | Pyrvinium Pamoate |
| Raloxifene | Ritanserin |
| RS 67333 HCl | RS 67506 HCl |
| Salmeterol | Sertindole |
| Sertraline | SKF-525A HCl |
| Tamoxifen Citrate | Tegaserod Maleate |
| Terbinafine HCl | Terconazole |
| Thioridazine | Toremifene Citrate |
| TMB-8 HCl | Trifluperidol HCl |
| Trifluoperazine HCl | Trimeprazine Hemi-L-Tartrate |
| Triflupromazine HCl | Tripelennamine HCl |
| Trimipramine Maleate | Verapamil HC |
| U-50488 HCl, (-)- | Xylazine |
| WB-4101 HCl | |

13. Use according to any or claims 1 to 12, **characterized in that** at least one chemotherapeutic agent used in the chemotherapy is selected from a platin-derivative, a vinca alkaloid or a taxane.

14. Use according to claim 13 **characterized in that** at least one chemotherapeutic agent used in the chemotherapy is selected from the group consisting of cisplatin, carboplatin and oxaliplatin; vincristine, vinblastine, vinorelbine and vindesine; paclitaxel and docetaxel.

15. Use according to claim 1 for the treatment or prevention of pain developing as a consequence of chemotherapy, wherein the compound binding to the sigma receptor is combined with a a least one chemotherapeutic drug (B) forming a fixed-dose active substance combination.

16. Use according to claim 15, wherein the chemotherapeutic drug (B) is selected from a platin-derivative, a vinca alkaloid or a taxane, especially the chemotherapeutic drug (B) is selected from is selected from cisplatin, carboplatin and oxaliplatin; vincristine, vinblastine, vinorelbine and vindesine; paclitaxel and docetaxel.
